# EUROPEAN PATENT APPLICATION

(11) **EP 4 438 716 A1**
(43) Date of publication of application: **02.10.2024**
(21) Application number: 23164815.5
(22) Date of filing: 28.03.2023
(51) Int. Cl.: C12N 1/20, C12P 19/04, C12P 21/02

(54) **IMPROVING MICROBIAL ACTIVITY**

(71) Applicant: BASF SE, 67056 Ludwigshafen am Rhein (DE)
(72) Inventor: May, Tobias, 67056 Ludwigshafen am Rhein (DE); Heuschkel, Ingeborg, 67056 Ludwigshafen am Rhein (DE); Haas, Evangeline Priya, 67056 Ludwigshafen am Rhein (DE); Heinrich, Daniel Christoph, 67056 Ludwigshafen am Rhein (DE); Herold, Andrea, 67056 Ludwigshafen am Rhein (DE)
(74) Representative: BASF IP Association

(57) **Abstract**

The invention is concerned with microorganisms, materials and methods for improving microbial activity. In particular, the invention provides methods for preparing microorganisms to improve metabolic activity and/or alignment, metabolically activated and/or aligned microorganisms, and methods of using them, in particular in industrial fermentation processes.

## Description

The invention is concerned with microorganisms, materials and methods for improving microbial activity. In particular, the invention provides methods for preparing microorganisms to improve metabolic activity and/or alignment, metabolically activated and/or aligned microorganisms, and methods of using them, in particular in industrial fermentation processes.

### BACKGROUND OF THE INVENTION

The goal of industrial fermentation processes typically is to produce, in the shortest possible time, a desired product using least expensive resources. In such processes, a microorganism culture or cell-free extract converts one, or typically more, metabolically accessible nutrients into a desired product. Typical industrial fermentation processes are submerged fermentations. In such processes, the microorganism culture is suspended in a fermentation medium which typically provides all nutrients required for production of the desired product; gaseous nutrients are typically provided using bubble columns or other forms of aeration.

Fermentation processes can be conducted as batch fermentations or continuous fermentations. In a batch fermentation, a fermentation medium is inoculated with a production microorganism seed culture. For the actual fermentation, the inoculated medium is then treated in a fermenter under conditions conductive to the production of the desired product. At the end of the fermentation, the fermentation medium comprising the desired product is harvested. Typically, the fermenter is then cleaned and prepared for another fermentation batch. A fed-batch fermentation differs from a batch fermentation by the addition of further substances (feed) during the fermentation. The feed may or may not have the same composition as the fermentation medium at the start of the fermentation. In a continuous fermentation, the whole or a sufficient part of the microorganism culture is retained in the fermenter, wherein the fermenter, continuously or intermittently, is fed with new feed and parts of the product-containing fermenter contents are harvested (partial harvest or repeated batch process).

Growth of microorganisms during fermentation typically passes through several stages. During lag phase (cf. Monod, Ann. Rev. Microbiol 1949, 371-394) after inoculation, the doubling time of a microorganism culture is high. It is thought that microorganisms need time to germinate and may adapt their metabolism to the change in medium composition. Lag phase is followed by a phase of exponential growth ("log phase"), wherein cell division proceeds at an approximately constant and high rate. For example, doubling times as short as 20 mins have been observed for Salmonella enterica during log phase. During exponential growth, microbial nutrient consumption rate is highest. It is thought that not all nutrients are consumed at all times at the same speed. Instead, some complex nutrients are thought to require the synthesis of specialised enzymes for consumption ("diauxic growth", cf. Salvy et al., Proc. Natl. Acad. Sci. USA 2021, doi: 1 0.1 073/pnas.2013836118). This may result in one or more periods of temporarily reduced growth rate during which the required changes in enzyme concentrations take place. With even less easily consumable nutrients left, growth enters a stationary phase in which doubling time increases significantly again. It is thought that microorganisms in stationary phase enter a dormant stage with severely reduced metabolic activity and/or that microorganisms in stationary phase subsist on the remains of dead cells, such that the doubling rate of cells more or less equals the death rate. Once stationary phase cannot be maintained any longer, the microorganism culture enters a decline stage in which the death rate is higher than the doubling rate.

In industrial fermentations it is desirable that microorganisms enter exponential phase as soon as possible and for as long as possible to maximize product formation. Bertrand et al. (J. Bacteriol. 2019, https://doi.org/10.1128/JB.00697-18) have reviewed factors influencing lag phase duration. They found that duration of lag phase is influenced not only by the history of the culture used for inoculation but also by the number of bacteria in a culture. As the number of cells increases, lag phase duration decreases. Caipo et al. (J. App. Microbiology 2002, 879-884) tested the effect of varying inoculum sizes (10E3 to 10E8 spores/ml) of a spore cell bank of Bacillus megaterium by microscopic evaluations. Their results indicate that higher concentrations of Bacillus megaterium spores encourage more rapid germination and more spores to germinate. According to the authors this is consistent with a theory by Woese et al. (Proc. Natl. Acad Sci USA 1968, 869-875), according to which Bacillus spores may contain a varying number of "germination molecules", such that at higher Bacillus spore densities a greater percentage of rapidly germinating spores will be present. Thus, a higher inoculum size is positively correlated with a faster germination. As completion of germination is a precondition for entering exponential growth for sporulated microorganisms, a large inoculum size is considered beneficial for industrial fermentations. Likewise, Webb et al. (Food Microbiol. 2012, 104-109) observe that inoculation spore concentration significantly affected germination time of Clostridium botulinum; decreasing the size of the C. botulinum spore inoculum increased time to toxin formation in food products. The authors propose that molecules from germinated spores could trigger germination of dormant neighbours.

Inoculation of the fermenter is typically performed by preparing a starter or seed culture of the production microorganism and adding a defined quantity thereof to the fermentation medium in the fermenter or preculture vessel. The seed culture is prepared by inoculating a volume of a cell bank vial or colonies from an agar-plate culture into a seed medium which may or may not have the identical composition as the fermentation medium and then incubating the inoculated seed medium under conditions suitable for growth of the microorganism culture. Typically, the fermentation medium is inoculated with a seed culture volume of 2-10% v/v of the fermenter volume (cf. WO2006096164 and WO2017068012).

Attempts have been made to obviate the seed culture preparation step. For example, WO2006096164 describes a fermentation of Clostridium botulinum for botulinum toxin production wherein the step of seed culture preparation is omitted such that the fermenter is inoculated directly with the contents of the cell bank vial. No positive effects have been attributed to such inoculation scheme, and the authors prefer approximately 2-4% of a seed medium having C. botulinum from a seed growth phase to be used to inoculate the fermentation medium. Likewise, WO2017068012 describes a fermentation of Trichoderma reesei for beta-xylosidase production, wherein the fermenter is inoculated directly from the contents of a cell bank vial or from the surface of a PDA agar plate. The authors speculate that such an inoculation scheme could be used not only for Ascomycota but also for bacteria. However, no data are shown in support of this speculation, and in preferred embodiments the microorganism is a filamentous fungus.

Quality of an industrial fermentation process does not only depend on lag phase duration but also on the yield of the desired product and the degree of formation of unwanted side products, e.g. slime or foam. Furthermore, reproducibility of fermentation results is required to avoid occurrence of surprisingly long lag phase duration, surprisingly short log phase duration or unforeseen nutrient demands.

It was thus the object of the present invention to provide improved materials and methods for industrial fermentations, in particular in relation to microorganism of family Paenibacillaceae.

### SUMMARY OF THE INVENTION

The invention provides a microorganism culture, wherein the microorganism is of family Paenibacillaceae, preferably of genus Paenibacillus, wherein the cells in the culture are
- metabolically aligned and/or
- in an activated metabolic state.

The invention also provides a fermentation method, comprising
i) providing a fermentation broth comprising a microorganism culture according to the invention in a fermentation medium, and
ii) cultivating the fermentation broth under conditions suitable to produce a target substance, and
iii) preferably recovering the target substance.

Furthermore, the invention provides a Method for reducing fermentation time required for producing a desired amount of a target substance, and/or for reducing viscosity during fermentation, and/or for increasing fermentation repeat accuracy, comprising the steps of
i) providing, in a fermentation medium, a culture according to the invention, and
ii) cultivating the culture in the fermentation medium.

And the invention provides a use of a culture according to the invention for reducing fermentation time required for producing a desired amount of a target substance, and/or for reducing viscosity during fermentation, and/or for increasing fermentation repeat accuracy.

The invention also provides a cultivation parameter dataset for controlling a fermentation, comprising
- a designation of a set of applicable fermentation media,
- a designation of a set of applicable samples of microorganisms,
- a designation of fermentation conditions conducive to obtain a culture according to the invention by cultivating an applicable sample in an applicable fermentation medium, wherein the fermentation conditions preferably comprise at least one of: type of cultivation medium, amount of cultivation medium, desired cultivation temperature range, desired cultivation aeration parameter ranges, desired amount of produced cells, desired duration of cultivation, desired pH during cultivation, desired yield or amount of a target substance.

And the invention provides a computer-implemented fermentation method, comprising
- receiving, by a computing device, a cultivation parameter dataset according to the invention,
- receiving a fermentation medium conforming to the cultivation parameter dataset,
- receiving a volume of a sample of microorganisms conforming to the cultivation parameter dataset, and
- controlling, by the computing device, a fermenter to cultivate said microorganisms and/or spores thereof in the fermentation medium under the fermentation conditions of the cultivation parameter dataset.

Furthermore, the invention provides a method for producing one or more viscosity-increasing substances, comprising
i) inoculating a fermentation medium by a concentration of a microorganism culture according to the invention in a predetermined concentration sufficient to obtain, during cultivation, a desired viscosity, and
ii) cultivating the culture obtained in step i), and
iii) preferably recovering the one or more viscosity-increasing or viscosifying substances.

### DESCRIPTION OF FIGURES

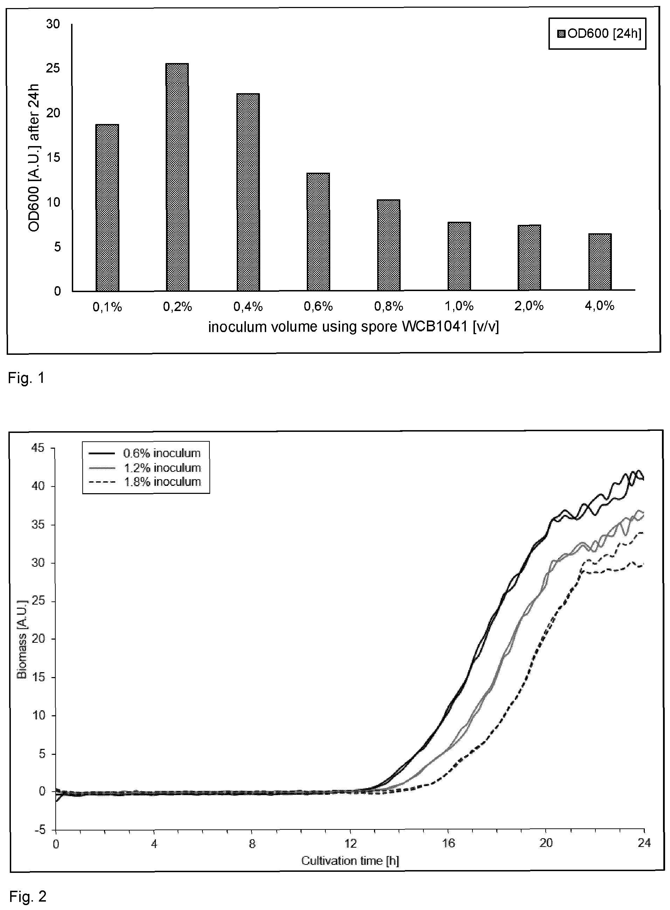

Fig. 1 shows the impact of inoculation volume on growth of Paenibacillus polymyxa LU21132 in shake flasks. Biomass formation is shown as OD600 [A.U.] after 24h cultivation time in shake flasks containing PX-125 complex medium. A range of different volumes of the working cell bank (WCB) 1041 was used for inoculation (0.1 - 4% v/v of total culture volume, as listed in table 1) of the culture media. With increasing amount of cells used for inoculation (i.e. increasing volume of WCB spore composition), optical density at 24h after inoculation decreases, indicating a slower growth of densely inoculated cultivation broths. Fastest growth is obtained at inoculation concentrations of 0.2-0.4% (v/v), at lower concentrations growth speed is again reduced.
Fig. 2 shows the impact of inoculation volume on growth of P.polymyxa LU20754 in 2ml deep well plates. Cultivation was carried out in a BioLector device (m2p labs, Germany) using a deep well plate with 48 positions at 900 rpm shaking frequency. Biomass formation was assessed periodically by scattered light measurement at 620 nm ex/em. Different volumes of a spore working cell bank from strain LU20754 were used for inoculation (0.6 - 1.8% v/v of total culture volume). The higher the inoculum concentration, the longer the delay before biomass reaches 20 A.U.

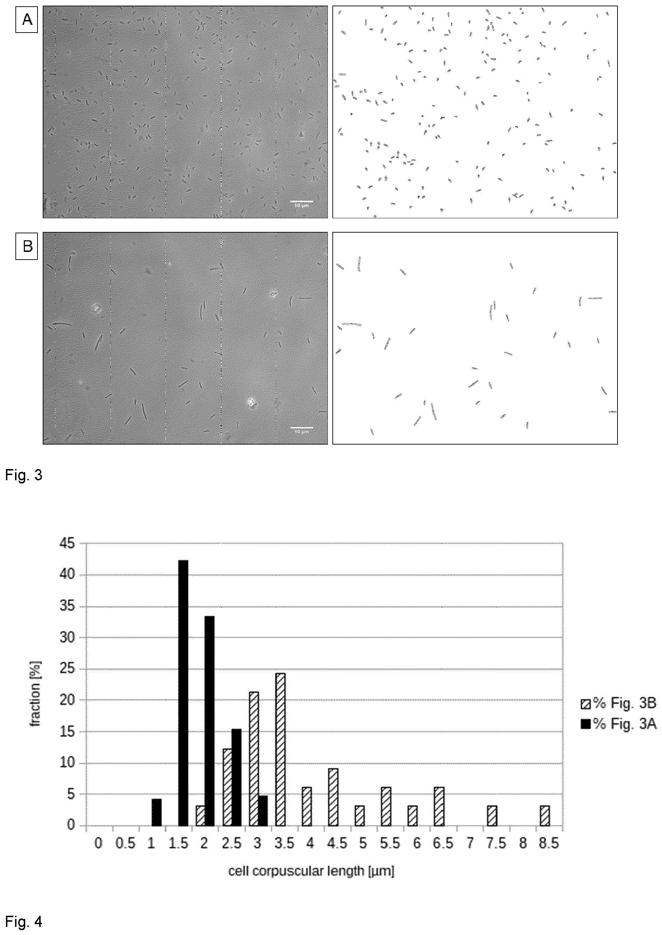

Fig. 3 shows microscopic pictures (left) and particle outlines (right) deduced thereof from shake flask cultures of P.polymyxa LU21552. Pictures were taken after 28h cultivation time. Shake flasks were inoculated with 0.2% v/v (fig. 3A) and 1% v/v (fig. 3B) inoculum volume using spore WCB1059 (prepared as described in table 5).
Fig. 4 shows the cell corpuscular lengths as determined from the particle outlines of figures 3A and 3B.

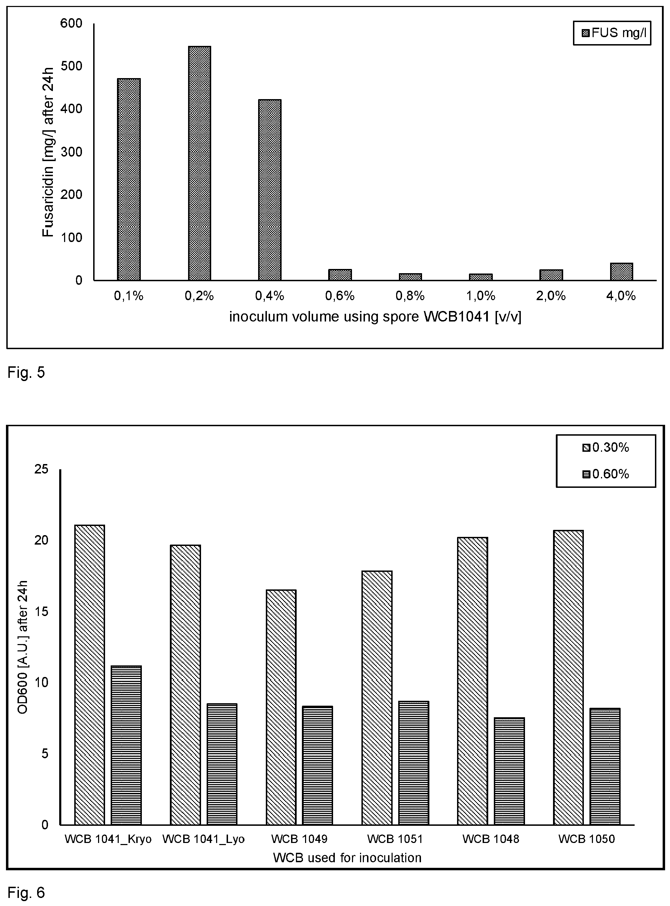

Fig. 5 shows the impact of shake flask inoculation volume on fusaricidin production of P.polymyxa LU21132. Fusaricidin titre is shown after 24h cultivation time in shake flasks containing PX-125 complex medium and using different working cell bank (WCB) inoculation volumes (0.1 - 4% v/v of total culture volume, table 1) of working cell bank WCB1041. The WCB1041 consists of 1.6E+08 (endo)spores/ml [cv% 9.5]. Cultivation conditions: 250ml shake flasks w/ baffles, 30ml media, 150 rpm, 2.5cm throw, 33°C, pH 6.5, 24h. At inoculation volumes of more than 0.4% (v/v), Fusaricidin yield sharply drops by a factor of roughly 50fold compared to the fusaricidin yield obtained at 0.2% (v/v) inoculation volume.
Fig. 6 shows the impact of shake flask inoculation volume on growth of P.polymyxa LU21132 using working cell banks (WCB) from six different preparation procedures as described in table 5. Biomass formation is shown as OD600 [A.U.] after 24h cultivation time in shake flasks containing PX-125 complex medium. Two different spore WCB inoculation volumes (0.3 and 0.6% v/v of total culture media volume) were used to test the growth properties of each of the six working cell banks. Cultivation conditions: 250ml shake flasks w/ baffles, 30ml media, 150 rpm, 2.5cm throw, 33°C, pH 6.5, 24h. Independent of working cell bank preparation, growth rate as indicated by the optical density at 600nm 24h after inoculation at 0.3% (v/v) is approximately twice as has as for corresponding cultivations inoculated at 0.6% (v/v).

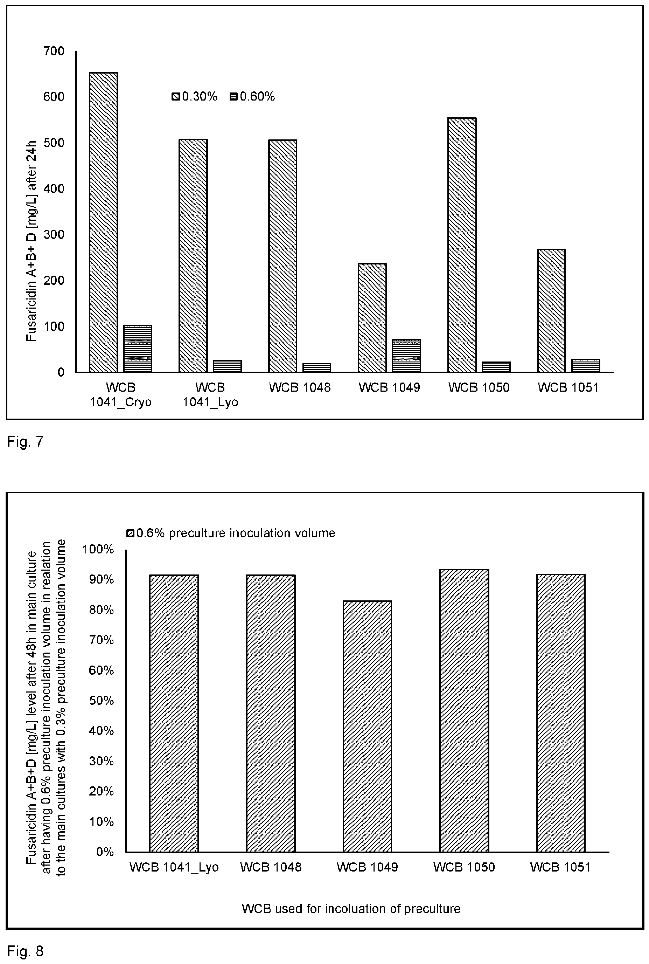

Fig. 7 shows the impact of shake flask inoculation volume on fusaricidin production of P.polymyxa LU21132 using six different working cell banks (WCB) which were prepared under varying conditions (described in table 5). Fusaricidin titre is shown at 24h cultivation time in shake flasks containing PX-125 complex medium and using two WCB inoculation volumes (0.3 and 0.6% v/v of total culture volume). Cultivation conditions: 250ml shake flasks w/ baffles, 30ml media, 150 rpm, 2.5cm throw, 33°C, pH 6.5, 24h. Independent of working cell bank preparation, fusaricidin tires for cultures inoculated by 0.3% (v/v) are approximately 3-25 times higher than for cultures inoculated by 0.6% (v/v) WCB spore composition.
Fig. 8 shows that initial preculture shake flask inoculation volume does affect the fusaricidin production in the main cultivation in a two-stage process: Fusaricidin A, B and D levels in shake flask main cultures with PX-143 media as a function of the preculture inoculation volume. Preculture flasks were inoculated with 0.3 and 0.6% (v/v) of different WCB types (see table 5). Cultures from example 3 were transferred after 24h cultivation time to main culture flasks using 2% (v/v) inoculation volume. The fusaricidin level of the main cultures that were inoculated with the preculture flasks having 0.6% (v/v) preculture inoculation volume are shown in relation to the fusaricidin level detected in the parallel main cultures from the 0.3% preculture inoculation variant. Cultivation conditions: 250ml shake flasks w/ baffles, 30ml media, 150 rpm, 2.5cm throw, 33°C, pH 6.5, 24h preculture, 48h main culture. Fusaricin content accumulated within 48h of fermentation is roughly 10% less in fermentations inoculated with 2% (v/v) of a pre-culture inoculated with 0.6% (v/v) WCB spore composition compared to corresponding fermentations inoculated with 2% (v/v) of a pre-culture inoculated with 0.3% (v/v) WCB spore composition (whose absolute fusaricidin content was roughly the same regardless of the WCB preparation and in each case was higher than 2000 mg/l).

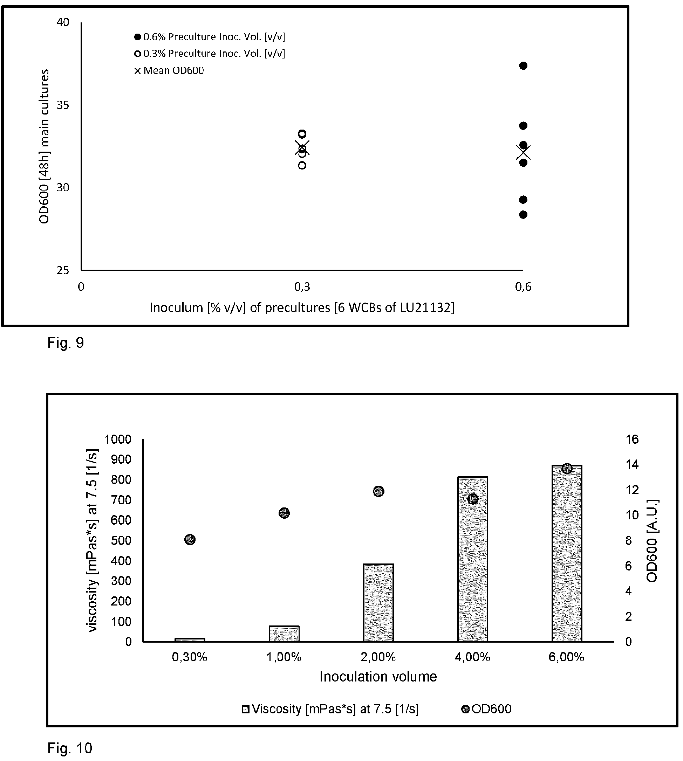

Fig. 9 shows that a lower preculture inoculum volume does synchronize growth in main culture (two stage process). The effect is demonstrated for six different working cell banks (WCB) of P.polymyxa LU21132 (prepared according to table 5). Preculture flasks (PX-125 media) were inoculated with 0.3 and 0.6% (v/v) of different WCB types of P.polymyxa LU21132 from table 5. Precultures were transferred after 24h cultivation time to main culture flasks (PX-143 media) using 2% (v/v) inoculation volume. OD600 (A.U.) of each cultivation from the six WCBs of table 5 was measured after 48h cultivation time in main culture media. Cultivation conditions: 250ml shake flasks w/ baffles, 30ml media, 150 rpm, 2.5cm throw, 33°C, pH 6.5, 24h preculture, 48h main culture.
Fig. 10 shows that main culture inoculation volume determines culture broth viscosity in soy-based medium (EPS-03) in shake flask cultivations (250ml shake flasks w/ baffles, 30ml media, 150 rpm, 2.5cm throw, 33°C, pH 6.5). Viscosity was assessed after 16h cultivation time. As indicated by OD600, EPS formation is in part decoupled from growth, with a special emphasis on 2, 4 and 6% inoculation volume.

### DETAILED DESCRIPTION OF THE INVENTION

The technical teaching of the invention is expressed herein using the means of language, in particular by use of scientific and technical terms. However, the skilled person understands that the means of language, detailed and precise as they may be, can only approximate the full content of the technical teaching, if only because there are multiple ways of expressing a teaching, each necessarily failing to completely express all conceptual connections, as each expression necessarily must come to an end. With this in mind, the skilled person understands that the subject matter of the invention is the sum of the individual technical concepts signified herein or expressed, necessarily in a pars-pro-toto way, by the innate constrains of a written description. In particular, the skilled person will understand that the signification of individual technical concepts is done herein as an abbreviation of spelling out each possible combination of concepts as far as technically sensible, such that for example the disclosure of three concepts or embodiments A, B and C are a shorthand notation of the concepts A+B, A+C, B+C, A+B+C. In particular, fallback positions for features are described herein in terms of lists of converging alternatives or instantiations. Unless stated otherwise, the invention described herein comprises any combination of such alternatives. The choice of more or less preferred elements from such lists is part of the invention and is due to the skilled person's preference for a minimum degree of realization of the advantage or advantages conveyed by the respective features. Such multiple combined instantiations represent the adequately preferred form(s) of the invention.

In so far as references are made herein to databases entries, e.g., Uniprot entries, the entries are those as published on 2022-01-10 10:00 CET. This also applies to sequences published under the corresponding database entry identifiers.

As used herein, terms in the singular and the singular forms like "a", "an" and "the" include plural referents unless the content clearly dictates otherwise. Thus, for example, use of the term "a nucleic acid" optionally includes, as a practical matter, many copies of that nucleic acid molecule; similarly, the term "probe" optionally (and typically) encompasses many similar or identical probe molecules. Also as used herein, the word "comprising" or variations such as "comprises" or "comprising" will be understood to imply the inclusion of a stated element, integer or step, or group of elements, integers or steps, but not the exclusion of any other element, integer or step, or group of elements, integers or steps.

As used herein, the term "and/or" refers to and encompasses any and all possible combinations of one or more of the associated listed items, as well as the lack of combinations when interpreted in the alternative ("or"). The term "comprising" also encompasses the term "consisting of".

The term "about", when used in reference to a measurable value, for example an amount of mass, dose, time, temperature, sequence identity and the like, refers to a variation of ± 0.1%, 0.25%, 0.5%, 0.75%, 1%, 2%, 3%, 4%, 5%, 6%, 7%, 8%, 9%, 10%, 15% or even 20% of the specified value as well as the specified value. Thus, if a given composition is described as comprising "about 50% X," it is to be understood that, in some embodiments, the composition comprises 50% X whilst in other embodiments it may comprise anywhere from 40% to 60% X (i.e., 50% ± 10%).

The term "microorganism" according to the invention denotes both individual microorganism cells and pluralities of such cells, the latter also being called "population" or, when being combined with a medium, a "culture". Preferably, the microorganism is a single taxonomic species and even more preferably is a single strain of a species. Thus, microbiologically pure cultures are preferred according to the present invention. Microorganism populations can also be consortia of microorganism cells of different strains of a common species and/or can be consortia comprising different taxonomic species or genera. Such mixed populations and cultures are, however, less preferred. The microorganisms may in principle be any kind of cells of taxonomic family Paenibacillaceae. The microorganism may be a wild-type organism, i.e. a microorganism that is essentially unaltered from the organism that originally was isolated from nature, or may be a modified microorganism, that has been modified by one or more technical procedures such as mutagenesis. Wild type microorganism samples preferably are recorded as type strains in culture collections. Genetic modification can be effected by random mutagenesis, for example NTG chemical mutagenesis, UV irradiation or transposon mutagenesis, or by directed mutagenesis, e.g. incorporation of heterologous plasmids or homologous recombination with heterologous nucleic acids and/or by site directed mutagenesis, e.g. using meganucleases, TALEN or CRISPR-type mutagenesis. For example, preferred methods of Bacillus and Paenibacillus mutagenesis are described in WO2017117395, incorporated herein in its entirety.

The microorganism used throughout the invention is of taxonomic family Paenibacillaceae. Microorganisms of taxonomic family Paenibacillaceae are defined by their 16S rDNA similarity (De Vos, P., W. Ludwig, K. H. Scheifer and W. B. Whitman 2009. Family IV. "Paenibacillaceae". Bergey's Manual of Systematic Bacteriology. P. De Vos, G. M. Garrity, D. Jones et al. New York, Springer. 3: 269). Their genome size typically is 3-9Mb. They are usually small to medium straight or curved rods, about 0.5-1.0 x 2-6 µm. They typically have Gram-positive wall structure and also typically are capable of producing spores. Members of this family have been isolated in various habitats ranging from Alaska to Antarctica in a variety of habitats, including deep sea sediment, ice cores, desert brines, sandstone biofilms, grassland soil, forest soil and agronomic plant roots. Due to the diversity of this family, it is expected that taxonomic rearrangements may happen in the future. Genera and species preferred according to the invention are described below in more detail.

Preferably the microorganism is of any of the genera Ammoniibacillus, Ammoniphilus, Aneurinibacillus, Oxalophagus, Brevibacillus, Candidatus Reconcilbacillus, Chengkuizengella, Cohnella, Fontibacillus, Gorillibacterium, Longirhabdus, Marinicrinis, Paenibacillus, Paludirhabdus, Saccharibacillus, Thermobacillus or Xylanibacillus, more preferably of genus Paenibacillus.

Particularly preferred are microorganisms of one of the following species:
Paenibacillus species: P. abekawaensis, P. abyssi, P. aceris, P. aceti, P. aestuarii, P. agarexedens, P. agaridevorans, P. alba, P. albidus, P. albus, P. alginolyticus, P. algorifonticola, P. alkaliterrae, P. alvei, P. amylolyticus, P. anaericanus, P. antarcticus, P. antibioticophila, P. antri, P. apiaries, P. apiarius, P. apis, P. aquistagni, P. arachidis, P. arcticus, P. assamensis, P. aurantiacus, P. azoreducens, P. azotifigens, P. baekrokdamisoli, P. barcinonensis, P. barengoltzii, P. beijingensis, P. borealis, P. bouchesdurhonensis, P. bovis, P. brasilensis, P. brassicae, P. bryophyllum, P. caespitis, P. camelliae, P. camerounensis, P. campinasensis, P. castaneae, P. catalpae, P. cathormii, P. cavernae, P. cellulosilyticus, P. cellulositrophicus, P. chartarius, P. chibensis, P. chinensis, P. chinjuensis, P. chitinolyticus, P. chondroitinus, P. chungangensis, P. cineris, P. cisolokensis, P. contaminans, P. cookii, P. crassostreae, P. cucumis, P. curdlanolyticus, P. daejeonensis, P. dakarensis, P. darangshiensis, P. darwinianus, P. dauci, P. dendritiformis, P. dongdonensis, P. donghaensis, P. doosanensis, P. durus, P. edaphicus, P. ehimensis, P. elgii, P. elymi, P. endophyticus, P. enshidis, P. esterisolvens, P. etheri, P. eucommiae, P. faecis, P. favisporus, P. ferrarius, P. filicis, P. flagellatus, P. fonticola, P. forsythiae, P. frigoriresistens, P. fujiensis, P. fukuinensis, P. gansuensis, P. gelatinilyticus, P. ginsengagri, P. ginsengarvi, P. ginsengihumi, P. ginsengiterrae, P. glacialis, P. glebae, P. glucanolyticus, P. glycanilyticus, P. gorillae, P. graminis, P. granivorans, P. guangzhouensis, P. harenae, P. helianthi, P. hemerocallicola, P. herberti, P. hispanicus, P. hodogayensis, P. hordei, P. horti, P. humicus, P. hunanensis, P. ihbetae, P. ihuae, P. ihumii, P. illinoisensis, P. insulae, P. intestini, P. jamilae, P. jilunlii, P. kobensis, P. koleovorans, P. konkukensis, P. konsidensis, P. koreensis, P. kribbensis, P. kyungheensis, P. lactis, P. lacus, P. larvae, P. lautus, P. lemnae, P. lentimorbus, P. lentus, P. liaoningensis, P. limicola, P. lupini, P. luteus, P. lutimineralis, P. macerans, P. macquariensis, P. marchantiophytorum, P. marinisediminis, P. marinum, P. massiliensis, P. maysiensis, P. medicaginis, P. mendelii, P. mesophilus, P. methanolicus, P. mobilis, P. montanisoli, P. montaniterrae, P. motobuensis, P. mucilaginosus, P. nanensis, P. naphthalenovorans, P. nasutitermitis, P. nebraskensis, P. nematophilus, P. nicotianae, P. nuruki, P. oceanisediminis, P. odorifer, P. oenotherae, P. oralis, P. oryzae, P. oryzisoli, P. ottowii, P. ourofinensis, P. pabuli, P. paeoniae, P. panacihumi, P. panacisoli, P. panaciterrae, P. paridis, P. pasadenensis, P. pectinilyticus, P. peoriae, P. periandrae, P. phocaensis, P. phoenicis, P. phyllosphaerae, P. physcomitrellae, P. pini, P. pinihumi, P. pinisoli, P. pinistramenti, P. pocheonensis, P. polymyxa, P. polysaccharolyticus, P. popilliae, P. populi, P. profundus, P. prosopidis, P. protaetiae, P. provencensis, P. psychroresistens, P. pueri, P. puernese, P. puldeungensis, P. purispatii, P. qingshengii, P. qinlingensis, P. quercus, P. radicis, P. relictisesami, P. residui, P. rhizoplanae, P. rhizoryzae, P. rhizosphaerae, P. rigui, P. ripae, P. rubinfantis, P. ruminocola, P. sabinae, P. sacheonensis, P. salinicaeni, P. sanguinis, P. sediminis, P. segetis, P. selenii, P. selenitireducens, P. senegalensis, P. senegalimassiliensis, P. seodonensis, P. septentrionalis, P. sepulcri, P. shenyangensis, P. shirakamiensis, P. shunpengii, P. siamensis, P. silagei, P. silvae, P. sinopodophylli, P. solanacearum, P. solani, P. soli, P. sonchi group, P. sophorae, P. spiritus, P. sputi, P. stellifer, P. susongensis, P. swuensis, P. taichungensis, P. taihuensis, P. taiwanensis, P. taohuashanense, P. tarimensis, P. telluris, P. tepidiphilus, P. terrae, P. terreus, P. terrigena, P. tezpurensis, P. thailandensis, P. thermoaerophilus, P. thermophilus, P. thiaminolyticus, P. tianmuensis, P. tibetensis, P. timonensis, P. translucens, P. tritici, P. triticisoli, P. tuaregi, P. tumbae, P. tundrae, P. turicensis, P. tylopili, P. typhae, P. tyrfis, P. uliginis, P. urinalis, P. validus, P. velaei, P. vini, P. vortex, P. vorticalis, P. vulneris, P. wenxiniae, P. whitsoniae, P. wooponensis, P. woosongensis, P. wulumuqiensis, P. wynnii, P. xanthanilyticus, P. xanthinilyticus, P. xerothermodurans, P. xinjiangensis, P. xylanexedens, P. xylaniclasticus, P. xylanilyticus, P. xylanisolvens, P. yanchengensis, P. yonginensis, P. yunnanensis, P. zanthoxyli, P. zeae, preferably P. agarexedens, P. agaridevorans, P. alginolyticus, P. alkaliterrae, P. alvei, P. amylolyticus, P. anaericanus, P. antarcticus, P. assamensis, P. azoreducens, P. barcinonensis, P. borealis, P. brasiliensis, P. brassicae, P. campinasensis, P. chinjuensis, P. chitinolyticus, P. chondroitinus, P. cineris, P. curdlanolyticus, P. daejeonensis, P. dendritiformis, P. ehimensis, P. elgii, P. favisporus, P. glucanolyticus, P. glycanilyticus, P. graminis, P. granivorans, P. hodogayensis, P. illinoisensis, P. jamilae, P. kobensis, P. koleovorans, P. koreensis, P. kribbensis, P. lactis, P. larvae, P. lautus, P. lentimorbus, P. macerans, P. macquariensis, P. massiliensis, P. mendelii, P. motobuensis, P. naphthalenovorans, P. nematophilus, P. odorifer, P. pabuli, P. peoriae, P. phoenicis, P. phyllosphaerae, P. polymyxa, P. popilliae, P. rhizosphaerae, P. sanguinis, P. stellifer, P. taichungensis, P. terrae, P. thiaminolyticus, P. timonensis, P. tylopili, P. turicensis, P. validus, P. vortex, P. vulneris, P. wynnii, P. xylanilyticus,
particularly preferred Paenibacillus peoriae, Paenibacillus jamilae, Paenibacillus brasiliensis, Paenibacillus kribbensis, Paenibacillus koreensis, Paenibacillus rhizosphaerae, Paenibacillus polymyxa, Paenibacillus amylolyticus, Paenibacillus terrae, Paenibacillus polymyxa polymyxa, Paenibacillus polymyxa plantarum, Paenibacillus nov. spec epiphyticus, Paenibacillus terrae, Paenibacillus macerans, Paenibacillus alvei,
more preferred Paenibacillus polymyxa, Paenibacillus polymyxa polymyxa, Paenibacillus polymyxa plantarum, Paenibacillus nov. spec epiphyticus, Paenibacillus terrae, Paenibacillus macerans, Paenibacillus alvei,
even more preferred Paenibacillus polymyxa, Paenibacillus polymyxa polymyxa, Paenibacillus polymyxa plantarum and Paenibacillus terrae.

Some suitable Paenibacillus strains are described and deposition information is given in the following international patent application; spores of such microorganisms or pesticidally active variants of any thereof can be incorporated as spores of the composition according to the invention: WO 2019155253: DSM32460 (Paenibacillus polymyxa strain VMC10/96), NCBI accession nr. NR_117743 (Paenibacillus peoriae DSM320), NCBI accession nr. HG324076 (Paenibacillus polymyxa DSM 36, clone 13), NCBI accession nr. AJ271157 (Paenibacillus jamilae CECT 5266), NCBI accession nr. NR_025169 (Paenibacillus kribbensis AM49), NCBI accession nr. NR_025106 (Paenibacillus brasiliensis PB172) , NCBI accession nr. AF391124 (Paenibacillus terrae AM141); WO2020181053: Paenibacillus sp. NRRL B-67721, Paenibacillus sp. NRRL B-67723, Paenibacillus sp. NRRL B-67724, Paenibacillus sp. NRRL B-50374

Most preferably the microorganism is a microorganism of taxonomic genus Paenibacillus and is selected from any of the species Paenibacillus polymyxa, Paenibacillus polymyxa polymyxa, Paenibacillus polymyxa plantarum and Paenibacillus terrae. According to the invention, the following type strains are preferred:

| species | Deposit |
|---|---|
| Paenibacillus abyssi | DSM 26238,CGMCC 1.12987 |
| Paenibacillus aestuarii | DSM 23861,JCM 15521,KACC 13125 |
| Paenibacillus aqarexedens | DSM 1327,CIP 107437 |
| Paenibacillus agaridevorans | DSM 1355,CIP 107436 |
| Paenibacillus alginolyticus | DSM 5050,NRRL-NRS 1347 |
| Paenibacillus algorifonticola | DSM 26746,JCM 16598,XJ259,CGMCC 1.10223 |
| Paenibacillus alkaliterrae | DSM 17040,KCTC 3956,CIP 109150 |
| Paenibacillus alvei | DSM 29,ATCC 6344,CCM 2051,IFO 3343,IMAB B-3-4,LMG 13253,NCIB 9371,NCTC 6352,NBRC 3343 |
| Paenibacillus amylolyticus | DSM 15211,NRRL NRS-290 |
| Paenibacillus anaericanus | DSM 15890,ATCC BAA-844,CIP 109447,KCTC 13749 |
| Paenibacillus apiarius | DSM 5581,ATCC 29575,NRRL-NRS 1438,NCIMB 13506 |
| Paenibacillus arcticus | DSM 108811,KCTC 33985,JCM 30981,MME2_R6,PAMC 28731 |
| Paenibacillus azoreducens | DSM 13822,NCIMB 13761 |
| Paenibacillus azotifigens | DSM 104138,KACC 18967,LMG 29963 |
| Paenibacillus azotofixans | DSM 5976,ATCC 35681,LMG 14658 |
| Paenibacillus barcinonensis | DSM 15478,CECT 7022 |
| Paenibacillus barengoltzii | DSM 22255,NBRC 101215,ATCC BAA-1209,CIP 109354 |
| Paenibacillus beijinqensis | DSM 24997,ACCC 03082 |
| Paenibacillus borealis | DSM 13188,CCUG 43137 |
| Paenibacillus bovis | DSM 28815,CGMCC 8333 |
| Paenibacillus brasilensis | DSM 14914,ATCC BAA-413,KCTC 3812,CIP 110686 |
| Paenibacillus brassicae | DSM 24983,ACCC 01125 |
| Paenibacillus campinasensis | DSM 21989,JCM 11200,KCTC 0364BP,BCRC 17341 |
| Paenibacillus castaneae | DSM 19417,CECT 7279 |
| Paenibacillus catalpae | DSM 24714,CGMCC 1.10784 |
| Paenibacillus cavernae | DSM 100100, KCTC 33652 |
| Paenibacillus cellulosilyticus | DSM 21372,CECT 5696,LMG 22232 |
| Paenibacillus chartarius | DSM 28439,CCUG 55240,CCM 7759 |
| Paenibacillus chibensis | DSM 11731,NRRL B-142,JCM 9905,IFO 15958,NBRC 15958 |
| Paenibacillus chinjuensis | DSM 15045,KCTC 8951,JCM 10939 |
| Paenibacillus chitinolyticus | DSM 11030,IFO 15660,NBRC 15660 |
| Paenibacillus chondroitinus | DSM 5051,NRRL-NRS 1351 |
| Paenibacillus chungangensis | DSM 28440,CCUG 59129,KCTC 13717 |
| Paenibacillus cineris | DSM 16945,CIP 108109,LMG 18439 |
| Paenibacillus cisolokensis | DSM 101873,UICC B-42,NRRL B-65368 |
| Paenibacillus cookii | DSM 16944,CIP 108110,LMG 18419 |
| Paenibacillus cucumis | DSM 101601,CCM 8655,LMG 29222 |
| Paenibacillus curdlanolyticus | DSM 10247,IFO 15724,NBRC 15724 |
| Paenibacillus daejeonensis | DSM 15491,KCTC 3745,JCM 11236 |
| Paenibacillus darwinianus | DSM 27245,ICMP 19912 |
| Paenibacillus dendritiformis | DSM 18844,CIP 105967 |
| Paenibacillus dongdonensis | DSM 27607,KCTC 33221 |
| Paenibacillus donghaensis | DSM 22278,LMG 23780,KCTC 13049 |
| Paenibacillus durus | DSM 1735,ATCC 27763,LMG 15707 |
| Paenibacillus ehimensis | DSM 11029,IFO 15659,NBRC 15659 |
| Paenibacillus elgii | DSM 22254,NBRC 100335,KCTC 10016BP,CIP 108552 |
| Paenibacillus elymi | DSM 106581,KCTC 33853 |
| Paenibacillus etheri | DSM 29760,CECT 8558 |
| Paenibacillus eucommiae | DSM 26048,KCTC 33054 |
| Paenibacillus faecis | DSM 23593,CIP 101062 |
| Paenibacillus favisporus | DSM 17253,LMG 20987,CECT 5760 |
| Paenibacillus filicis | DSM 23916,KCTC 13693,JCM 16417,KACC 14197 |
| Paenibacillus fonticola | DSM 21315,LMG 23577,BCRC 17579 |
| Paenibacillus forsythiae | DSM 17842,CCBAU 10203,CIP 110608 |
| Paenibacillus frigoriresistens | DSM 25554,JCM 18141,CCTCC AB 2011150 |
| Paenibacillus gansuensis | DSM 16968,KCTC 3950,CIP 109446 |
| Paenibacillus ginsengarvi | DSM 18677,KCTC 13059,CIP 109800 |
| Paenibacillus ginsengihumi | DSM 21568,JCM 14928,KCTC 13141 |
| Paenibacillus glucanolyticus | DSM 5162,ATCC 49278,IFO 15330,NCIB 12809,S93,NBRC 15330 |
| Paenibacillus glycanilyticus | DSM 17608,JCM 11221,CIP 107742,KCTC 3808,NRRL B-23455,DS-1 |
| Paenibacillus gorillae | DSM 26181,CSUR P205 |
| Paenibacillus graminis | DSM 15220,ATCC BAA-95,LMG 19080 |
| Paenibacillus harenae | DSM 16969,KCTC 3951 |
| Paenibacillus hodogayensis | DSM 22253,JCM 12520,KCTC 3919 |
| Paenibacillus hongkongensis | DSM 17642,CCUG 49571,CIP 107898 |
| Paenibacillus humicus | DSM 18784,NBRC 102415,LMG 23886,CIP 110609 |
| Paenibacillus hunanensis | DSM 22170,ACCC 10718,CGMCC 1.8907,CIP 110610 |
| Paenibacillus ihumii | DSM 100664,CSUR P1981,CSUR 1981 |
| Paenibacillus illinoisensis | DSM 11733,NRRL NRS-1356,JCM 9907,IFO 15959,NCIMB 13573,NBRC 15959 |
| Paenibacillus jilunlii | DSM 23019,CGMCC 1.10239,CIP110611 |
| Paenibacillus kobensis | DSM 10249,IFO 15729,NBRC 15729 |
| Paenibacillus konkukensis | DSM 104139,KACC 18876,LMG 29568 |
| Paenibacillus konsidensis | DSM 21992,JCM 14798,KCTC 13165 |
| Paenibacillus kribbensis | DSM 27933,JCM 11465,KCTC 0766BP |
| Paenibacillus lactis | DSM 15596,LMG 21940,CIP 108827 |
| Paenibacillus larvae | DSM 7030,ATCC 9545,LMG 9820,Med-540,NRRL B-2605 |
| Paenibacillus lautus | DSM 3035,ATCC 43898,LMG 11157,NCIMB 12780 |
| Paenibacillus lentus | DSM 25539,ATCC BAA-2594 |
| Paenibacillus macerans | DSM 24,ATCC 8244,CCM 2012,IAM 12467,LMG 13281,NCIB 9368,NCTC 6355,CIP 66.19 |
| Paenibacillus macquariensis subsp. defensor | DSM 23149,JCM 14954,NCIMB 14397 |
| Paenibacillus macquariensis subsp. macquariensis | DSM 2,ATCC 23464,LMG 6935,CIP 103269,NCTC 10419 |
| Paenibacillus marchantiophytorum | DSM 29850,CGMCC 1.15043 |
| Paenibacillus massiliensis subsp. massiliensis | DSM 16942,CIP 107939,CCUG 48215 |
| Paenibacillus mendelii | DSM 19248,CCM 4839,LMG 23002 |
| Paenibacillus motobuensis | DSM 18200,JCM 12774,CCUG 50090,GTC 1835 |
| Paenibacillus mucilaginosus | DSM 24461,CIP 105815,HSCC 1605,KCTC 3870,VKM B-1480D,VKPM B-7519 |
| Paenibacillus nanensis | DSM 22867,KCTC 13044,TISTR 1828,PCU 276 |
| Paenibacillus naphthalenovorans | DSM 14203,ATCC BAA-206 |
| Paenibacillus nebraskensis | DSM 103623,CIP 111179,LMG 29764 |
| Paenibacillus nematophilus | DSM 13559,CIP 108049 |
| Paenibacillus nicotianae | DSM 28018,NRRL B-59112,CGMCC 1.12819 |
| Paenibacillus odorifer | DSM 15391,ATCC BAA-93,LMG 19079 |
| Paenibacillus ottowii | DSM 107750,ATCC TSD-165 |
| Paenibacillus pabuli | DSM 3036,ATCC 43899,LMG 11158,NCIMB 12781 |
| Paenibacillus panacisoli | DSM 21345,KCTC 13020,LMG 23405 |
| Paenibacillus pasadenensis | DSM 19293,NBRC 101214,ATCC BAA-1211 |
| Paenibacillus pectinilyticus | DSM 28340,CECT 7358,KCTC 13222 |
| Paenibacillus peoriae | DSM 8320,LMG 14832,NRRL B-14750 |
| Paenibacillus phoenicis | DSM 27463,NBRC 106274,NRRL B-59348 |
| Paenibacillus phyllosphaerae | DSM 17399,LMG 22192,CECT 5862 |
| Paenibacillus physcomitrellae | DSM 29851,CGMCC 1.15044 |
| Paenibacillus pinihumi | DSM 23905,KCTC 13695,JCM 16419,KACC 14199 |
| Paenibacillus piri | DSM 105496,KACC 19385 |
| Paenibacillus pocheonensis | DSM 23906,KCTC 13941,LMG 23404 |
| Paenibacillus polymyxa | DSM 36,ATCC 842,BUCSAV 162,CCM 1459,JCM 2507,LMG 13294,NCIB 8158,NCTC 10343 |
| Paenibacillus popilliae | DSM 22700,CIP 106066,NRRL B-2309,ATCC 14706,CCUG 28881,NCCB 75017 |
| Paenibacillus prosopidis | DSM 22405,LMG 25259,CECT 7506 |
| Paenibacillus provencensis | DSM 22280,CCUG 53519,CIP 109358 |
| Paenibacillus pueri | DSM 22870,KCTC 13223,CECT 7360 |
| Paenibacillus puldeunqensis | DSM 27603,KCTC 13718,CCUG 59189 |
| Paenibacillus purispatii | DSM 22991,CIP 110057 |
| Paenibacillus qingshengii | DSM 100926,JCM 30613,CCTCC AB 2014290 |
| Paenibacillus radicis | DSM 100762,CGMCC 1.15286 |
| Paenibacillus relictisesami | DSM 25385,JCM 18068 |
| Paenibacillus residui | DSM 22072,CCUG 57263 |
| Paenibacillus rhizophilus | DSM 103168,CGMCC 1.15699 |
| Paenibacillus rhizoplanae | DSM 103963,LMG 29875,CCM 8725 |
| Paenibacillus rhizosphaerae | DSM 17254,LMG 21955,CECT 5831 |
| Paenibacillus ripae | DSM 104672,LMG 29834,CCTCC AB 2014276,LMG 28639 |
| Paenibacillus sabinae | DSM 17841,CCBAU 10202,CIP 109632,KCTC 13697 |
| Paenibacillus sacheonensis | DSM 23054,KACC 14895,CIP 110612 |
| Paenibacillus sanguinis | DSM 16941,CIP 107938,CCUG 48214 |
| Paenibacillus sediminis | DSM 23491,GT-H3,LMG 25635,CIP 110613 |
| Paenibacillus segetis | DSM 28014,CGMCC 1.12769 |
| Paenibacillus senegalensis | DSM 25958,CSUR P157 |
| Paenibacillus sepulcri | DSM 27042,LMG 19508,CCM 7311,Heyrman R-514,mcha6024 |
| Paenibacillus shirakamiensis | DSM 26806,NBRC 109471 , KCTC 33126,CIP 110571 |
| Paenibacillus silagei | DSM 101953,JCM 30974 |
| Paenibacillus silvae | DSM 28013,CGMCC 1.12770 |
| Paenibacillus solani | DSM 100999,CCTCC AB 2015207 |
| Paenibacillus soli | DSM 21316,LMG 23604,KCTC 13010 |
| Paenibacillus sonchi | DSM 28159,CECT 7330,CCGB 1313 |
| Paenibacillus sophorae | DSM 23020,CGMCC 1.10238,CIP 110614 |
| Paenibacillus sputi | DSM 22699,KCTC 13252 |
| Paenibacillus stellifer | DSM 14472 |
| Paenibacillus taichunqensis | DSM 19942,BCRC 17757,CIP 110615 |
| Paenibacillus taiwanensis | DSM 18679,IAM 15414,LMG 23799 |
| Paenibacillus taohuashanense | DSM 25809,CGMCC 1.12175 |
| Paenibacillus tarimensis | DSM 19409,CCTCC AB 206108,CIP 110616 |
| Paenibacillus terrae | JCM:11466, KCCM:41557 |
| Paenibacillus terreus | DSM 100035,KACC 18491,CCTCC AB 2015273 |
| Paenibacillus terrigena | DSM 21567,CCTCC AB206026,IAM 15291,JCM 21741 |
| Paenibacillus thailandensis | DSM 22866,KCTC 13043,TISTR 1827,PCU 275 |
| Paenibacillus thermoaerophilus | DSM 26310,JCM 18657 |
| Paenibacillus thiaminolyticus | DSM 7262,JCM 8360,NRRL B-4156 |
| Paenibacillus tianmuensis | DSM 22342,CGMCC 1.8946,CIP 110617 |
| Paenibacillus tibetensis | DSM 29321,ACCC 19728 |
| Paenibacillus timonensis | DSM 16943,CIP 108005,CCUG 48216 |
| Paenibacillus triticisoli | DSM 25425,CGMCC 1.12045 |
| Paenibacillus tundrae | DSM 21291,NRRL B-51094,CIP 110036 |
| Paenibacillus turicensis | DSM 14349,NCCB 100011 |
| Paenibacillus tylopili | DSM 18927,LMG 23975 |
| Paenibacillus typhae | DSM 25190,CGMCC 1.1 1012,CIP 110618 |
| Paenibacillus uliginis | DSM 21861,LMG 24790 |
| Paenibacillus urinalis | DSM 22281,CCUG 53521,CIP 109357 |
| Paenibacillus validus | DSM 3037,ATCC 43897,LMG 11161,NCIMB 12782,NRRL-NRS 1000 |
| Paenibacillus vulneris | DSM 27954,JCM 18268,CCUG 53270 |
| Paenibacillus wenxiniae | DSM 100576,CGMCC 1.15007 |
| Paenibacillus woosongensis | DSM 16971,KCTC 3953,CIP 110595 |
| Paenibacillus wulumuqiensis | DSM 29194,CPCC 100602,JCM 30284 |
| Paenibacillus wynnii | DSM 18334,CIP 108306,LMG 22176 |
| Paenibacillus xerothermodurans | DSM 520,ATCC 27380 |
| Paenibacillus xinjianqensis | DSM 16970,KCTC 3952,CIP 109466 |
| Paenibacillus xylanexedens | DSM 21292,NRRL B-51090,CIP 110619 |
| Paenibacillus xylaniclasticus | DSM 26531,NBRC 106381,KCTC 13719,TISTR 1914 |
| Paenibacillus xylanilyticus | DSM 17255,LMG 21957,CECT 5839 |
| Paenibacillus xylanisolvens | DSM 25299,KCTC 13042,PCU 311,TISTR 1829 |
| Paenibacillus xylanivorans | DSM 107920,NCIMB 15123 |
| Paenibacillus zanthoxyli | DSM 18202,CCBAU 10243,CIP 109595,KCTC 13696 |

As described above there is a widespread belief in the art, supported by evidence in several species throughout the Firmicutes microorganisms, that a high inoculation titre is beneficial for industrial fermentations, in particular to reduce lag phase duration and to ensure fast germination of spores. Contrary to this belief the inventors have found that a low inoculation titre is beneficial for industrial fermentations. At the same time the inventors found that very low inoculation titres, as suggested in for example WO2006096164 and WO2017068012, are not beneficial in Paenibacillaceae industrial fermentations. While useful inoculation titres will be described herein, in particular in the examples section, it is understood that optimal inoculation titres are influenced to some degree also by medium composition. The invention correspondingly provides a method of screening an inoculation quantity of a microorganism of family Paenibacillaceae, more preferably of genus Paenibacillus.

According to the invention, the term "spore", "spores", "endospore" and "endospores" are used interchangeably.

The invention provides a microorganism culture, wherein the microorganism is of family Paenibacillaceae, preferably of genus Paenibacillus. The term "culture" according to the invention means a collection or assemblage of microorganisms wherein at least 50% of all living cells and spores belong to the Paenibacillaceae family, preferably to genus Paenibacillus. More preferably, the culture is a pure culture of microorganisms belonging to the Paenibacillaceae family, preferably to genus Paenibacillus. Furthermore, a "culture" according to the present invention can be both a liquid culture, wherein microorganisms are suspended in a liquid medium suitable for their growth, or a preparation of microorganisms in a form suitable for inoculation of a liquid culture, preferably in the form of a cryoconservation cell bank vial, preferably containing a antifreezing and / or stabilizing agent, for example glycerol and / or a sugar. Media and methods for creating and maintaining cell bank vials are known to the skilled person.

The cells in the culture are metabolically aligned and/or in an activated metabolic state. It is a particular advantage of the present invention that such cultures are provided for the first time in industrially relevant quantities, that is, in liquid culture volumes of more than 10l, more preferably 10-1000 I, even more preferably 20-1000!, even more preferably 50-1000!, even more preferably 100-1000 I. However, it is a particular advantage of the method that the medium volume required for performing the method can be small, preferably 10-500 ml, more preferably 15-250 ml, even more preferably 20-100 ml. As described herein, the invention is advantageously applicable for industrial fermentations. Thus, the fermenter vessel preferably has a medium volume of more than 200l, preferably 300l, more preferably 400-1000!, more preferably 500l, more preferably 1000-150000!, more preferably 2000-150000l, more preferably 50000-150000!. It is a particular advantage that the fermenter can have a medium volume of 50-300m³, such fermenter volumes are frequently encountered in existing industrial fermentation plants. Thus, the Invention advantageously avoids a need for re-fitting, in particular up- or downsizing, of existing fermentation hardware.

Cultures of microorganism cells that are metabolically aligned and/or in an activated metabolic state have numerous advantages in fermentation processes as are described below in more detail. In particular, such cultures allow for reducing fermentation time required for producing a desired amount of a target substance, and/or for reducing viscosity during fermentation, and/or for increasing fermentation repeat accuracy. It is particularly astonishing that such advantages are obtainable by maintaining adequate conditions of cultivation of Paenibacillaceae microorganisms, particularly of species belonging to genus Paenibacillus. Such microorganisms have been used in diverse applications in the past. However, so far genetic modifications of such microorganisms have been considered to be the only feasible way to significantly reduce fermentation times required for producing a desired amount of a target substance, and/or to reduce viscosity during fermentation, and/or to increase fermentation repeat accuracy relative to a wild-type cell. It is a further advantage of the present invention that the required properties of a microorganism culture for seeding an industrial fermentation are easily observable using standard laboratory practice and equipment. Furthermore, the conditions for producing a microorganism culture according to the present invention are robust such that variations in operation typical for a laboratory or production environment can be tolerated. In particular the time between inoculating the growth medium and harvesting the culture can be varied by several hours. Thus, the properties that identify the microorganism culture are, in practice, easy to determine and can be reliably obtained using standard media and equipment.

In a culture of the present invention, the microorganisms of family Paenibacillaceae, preferably of genus Paenibacillus, are preferably characterised by any of the following parameters:
a) the median cell corpuscular length is 0.8-2.9 µm, more preferably 1-2.5 µm, even more preferably 1.3-2 µm, even more preferably 1.3-1.8µm, and/or
b) the 75^{th} cell corpuscular length percentile is 1.3-3 µm, more preferably 1.3-2.9 µm, more preferably 1.5-2.5 µm, and/or
c) the 25^{th} cell corpuscular length percentile is 0.8-2.5 µm, more preferably 0.8-1.9 µm, more preferably 0.8-1.5 µm.

Thus, 75% of the cell corpuscles preferably have a length of at most 3 µm, more preferably up to 2.9 µm, more preferably at most 2.5 µm, and/or 25% of the cell corpuscles have a length of at most 2.5µm, more preferably 1.9 µm, even more preferably 0.8-1.5 µm.

Microorganisms of family Paenibacillaceae form, in liquid culture, rod-like corpuscles. According to the invention, the "cell corpuscular length" is the extension, along the longest axis, of such corpuscle. The accompanying figures and examples depict such corpuscles in greater detail and describe methods for determining the cell corpuscular length. It is a particular advantage of the present invention that microorganisms of family Paenibacillaceae produce two clearly different growth types in liquid culture. In the common type observed in state of the art fermentations, cells are rather large with a median cell corpuscular length of 3.4µm. However, the inventors have found that a second growth type exists which offers the advantages of the present invention. Cells growing in this growth type are called herein "metabolically aligned" or "in an activated metabolic state", because their fermentation properties in liquid fermentations are significantly and consistently different from the properties of microorganisms growing in the first growth type as described herein. It is a particular advantage that microorganisms of the second growth type, the "metabolically aligned" microorganisms or microorganisms "in an activated metabolic state", can be told apart from those of the first growth type by simple microscopy. For a trained user, the transition from the first growth type to the second growth type in liquid cultivation is readily observable due to the profound change in median cell corpuscular length.

A Paenibacillaceae microorganism culture of the present invention is preferably obtainable or obtained by inoculating a cultivation medium with a concentration of microorganisms sufficient to produce said culture, wherein the concentration is preferably determined by
i) cultivating a series of cultures inoculated with different inoculation concentrations for a sufficient time to differentiate cultures by a parameter indicative of metabolic activity, wherein the parameter is preferably selected from any of growth rate, cell size, viscosity, production of an indicator substance, optical density, cell count, course of pH, gas formation, oxygen uptake rate, CO2 transfer rate, and variance of any of the aforementioned parameters, and
ii) determining said one or more parameter, and
iii) selecting an inoculation concentration based on the determined one or more parameters.

It is a particular advantage of the present invention that the transition from the first growth type to the second, metabolically aligned or activated growth type, can be induced by selecting a sufficiently low inoculation concentration. Inoculation can be performed by seeding a fresh growth medium with microorganism cells and/or spores. By varying the inoculation concentration, the optimal concentration can be found to achieve a transition from the first to the second growth type within a desired cultivation time.

It is a further advantage of the present invention that instead of cell corpuscular length also other parameters can be observed to indicate the transition to a culture of metabolically aligned microorganisms and/or microorganisms in an activated metabolic state. As described below in more detail, microorganism cultures of the present invention advantageously provide an increased metabolic activity compared to wild type cells in the first growth type. Preferably such increased metabolic activity is determined from any of growth rate, cell size, viscosity or production of an indicator substance, optical density, cell count, course of pH, gas formation, oxygen uptake rate and CO2 transfer rate. It is also a particular advantage that the microorganism culture of the present invention comprises metabolically aligned cells and thus allows to achieve highly consistent results in repeated fermentation. Thus, in addition to or instead of measuring any of the aforementioned parameters as such, variance of any of the aforementioned parameters can also be determined. A low variance indicates successful transition to the second growth type.

For example, production of an indicator substance is preferably determined by the concentration at a predetermined time or the time to reach a predetermined concentration. The indicator substance can be chosen at will. For example, the indicator substance could be a coloured substance, a metabolic side product (e.g. acetoine, 2,3 BDO, glutamate) or a target substance, preferably a fusaricidin, paeniserine, paeniprolixine or polymyxin. Thus, the batches are preferably compared by any of the following criteria:
- time required to achieve a predefined yield of a target substance, preferably a fusaricidin, paeniserine, paeniprolixine or polymyxin,
- yield of a target substance, preferably a fusaricidin, paeniserine, paeniprolixine or polymyxin at a predetermined time after start of step ii), preferably at 8-48h, more preferably 12-24h.

In view of the aforementioned advantages the invention also provides a fermentation method, comprising
i) providing a fermentation broth comprising a microorganism culture of the present invention in a fermentation medium, and
ii) cultivating the fermentation broth under conditions suitable to produce a target substance.

The fermentation method of the present invention offers surprising benefits compared to fermentation methods not using microorganisms of family Paenibacillaceae in an activated and/or aligned metabolic state: One advantage is an increased fermentation speed. In particular, the fermentation method allows to achieve a significantly shortened lag phase after transfer of the inoculum into the fermentation medium. Furthermore, the fermentation method lets microorganisms require less time to reach half-maximal cell and/or optical density. Also, the fermentation method allows to achieve increased productivity, i.e. yield per time, and total yield of target substances. For Paenibacillus microorganisms this allows to obtain an advantageously increased yield of fusaricidin, paeniserine, paeniprolixine and/or polymyxin. Furthermore, development of high viscosity can be advantageously delayed or controlled. This allows to avoid a high energy input for stirring of medium and facilitates purification of produced target substances.

Particularly advantageous and surprising is that the fermentation proceeds with high reproducibility. This is be ascertained preferably by a very low variability of optical density at any given time for repeated fermentations. Thus, the fermentation method according to the invention is particularly suitable for automation, because once a fermentation protocol is established, the need for interfering steering by a user to counter unforeseen divergent fermentation developments can be reduced or avoided altogether. Correspondingly, the fermentation method of the present invention allows to produce a target substance of reliable quality over several batches.

Another effect of the significantly increased reproducibility of fermentations is that variations in the growth and metabolic behaviour of microbes during fermentation caused by small variations in cell bank sample properties are avoided. Instead, by starting an industrial fermentation with a microorganism culture in a defined state, i.e. a microorganism culture of the present invention, shields the fermentation from such random disturbances.

In step i) the fermentation broth is preferably prepared by inoculating a cell free fermentation medium with a microorganism culture of the present invention. Thus, the fermentation medium is inoculated with Paenibacillaceae microorganisms, preferably with a pure culture of Paenibacillus microorganisms, which already are metabolically aligned and/or in an activated metabolic state. By using the microorganism culture of the present invention, only a short adaptation time is required before the microorganisms become productive for producing a target substance. Preferably, when producing the microorganism culture of the present invention by the above production method, the microorganism culture is already grown in the fermentation medium of step i) of the fermentation method. This is further suitable to decrease the adaptation time and to achieve a particularly high productivity, i.e. the amount of recoverable target substance produced in step ii) in a given time and fermentation volume is particularly high. The fermentation broth can also be prepared by inoculating a fermentation medium with purified cells of a microorganism culture of the present invention.

Preferably the fermentation medium is inoculated in step i) with a sufficient quantity of the microorganisms to sustain the metabolically aligned and/or activated state in step ii). It is a particular advantage of the present invention that optimising the inoculation quantity can be done by observing any of the aforementioned parameters growth, cell size, viscosity and production of an indicator substance and/or their variance. Typically for microorganisms of genus Paenibacillus in a metabolically aligned or activated state will be added to reach at least 2E4 cfu/ml in the fermentation medium in industrial fermentations. Preferably, a fermentation or cultivation medium is inoculated by 2E05 - 2E06 cfu/ml (200000 - 2000000 cfu/ml).

It is a particular advantage of the present invention that the microorganisms retain their metabolically aligned and/or activated state over several passages. For example, a microorganism culture of the present invention can be poured as such into a new fermentation medium for inoculation, or the cells of the microorganism culture can be purified, preferably by centrifugation, and be introduced to the new fermentation medium. Either way, the cells will continue to grow according to the second growth type as described herein. Thus, the invention avoids, for the inoculation of a large industrial fermentation volume, a necessity to prepare the microorganism population required in step i) in a single step, which would most likely have required storing a lot of inoculation material in cell banks. Instead, microorganisms of a cell bank vial can first be grown in a small shaking beaker liquid culture of approximately 50ml - 2500ml until they have transitioned to the second growth type and thus have reached a metabolically aligned and/or an activated metabolic state. Then, the cells are transferred (in the fermentation broth or after purification and preferably resuspension in new fermentation medium) to successively larger fermentation volumes.

In step ii) the fermentation broth is fermented under suitable conditions for producing a target substance. It is a particular advantage that the fermentation using the microorganism culture of the present invention is not limited to particular conditions. Instead, the suitable conditions can be chosen by the skilled person particularly in view of oxygen and nutrient demand, development of viscosity and/or production of a target substance. Furthermore, additional components may be added to or extracted from the fermentation medium. Preferably the pH of the fermentation broth is adjusted by addition of appropriate amounts of acids or bases. Preferably the oxygen concentration is adjusted by increasing or decreasing aeration, for example bubbling oxygen into the fermentation broth and/or adjusting the speed of a mixer. Temperature is preferably adjusted by heating or cooling the mantle of the fermenter vessel and/or by increasing or decreasing the concentration and/or type of available nutrients. Also preferably the medium comprises or is supplied during fermentation with an antifoaming agent in sufficient amount to prevent or reduce formation of foam during fermentation step ii).

Preferably the fermentation method of the present invention further comprises
iii) recovering the target substance from the cultivated fermentation broth of step ii).

The target substance according to the present invention preferably is selected from
- microorganisms, preferably viable cells and/or spores, and/or
- one or more metabolic products, preferably at least one fusaricidin, paeniserine, paeniprolixine or polymyxin, preferably at least two or more fusaricidins, paeniserines, paeniproxilines or polymyxins, more preferably 3 to 120 fusaricidins, wherein the one or more fusaricidins comprise any of fusaricidin A, B or D, and/or surfactin and/or iturin.

In particular, the target substance preferably is any of:
- a composition comprising spores and/or cells of the microorganism, preferably a composition comprising or consisting of viable cells and spores in a ratio of at most 4:1, more preferably 3:1 to 0.2:1, even more preferably less than 0.2:1, even more preferably only containing spores without viable cells,
- a composition comprising or consisting of at least one fusaricidin, paeniserine, paeniprolixine or polymyxin, preferably at least two or more fusaricidins, paeniserines, paeniproxilines or polymyxins, more preferably 3 to 120 fusaricidins, wherein the one or more fusaricidins comprise any of fusaricidin A, B or D, and/or surfactin and/or iturin, and/or
- a composition comprising or consisting of at least one viscosity increasing or viscosifying substance preferably selected from expopolysaccharides, proteoglycans and poly-gamma-glutamic acid.

It is a particular advantage of the present invention that the target substance is not limited to a particular type of substance. As shown in the examples, using a fermentation method of the present invention allows to reproducibly obtain higher productivity and/or higher yields for various target substances compared to a fermentation method wherein the fermentation medium is not inoculated using a population of metabolically aligned and/or activated microorganisms.

The target substance can be cells and/or spores of the microorganism. In agriculture, bacterial spores were used in plant pest control compositions reducing or preventing phytopathogenic fungal or bacterial diseases. Spore biologicals are also applied to improve plants resistance against biotic and abiotic stress, to accelerate the growth of the plant and to increase the yield during plant, fruit or legume harvest. Spore products were applied to leaves, shoots, fruits, roots or plant propagation material as well as to the substrate where the plants are to grow (Toyota K. Bacillus-related Spore Formers: Attractive Agents for Plant Growth Promotion. Microbes Environ. 2015;30(3):205-207. doi:10.1264/jsme2.me3003rh). Bochow, H., et al. "Use of Bacillus Subtilis as Biocontrol Agent. IV. Salt-Stress Tolerance Induction by Bacillus Subtilis FZB24 Seed Treatment in Tropical Vegetable Field Crops, and Its Mode of Action / Die Verwendung von Bacillus Subtilis zur biologischen Bekämpfung. IV. Induktion einer Salzstress-Toleranz durch Applikation von Bacillus subtilis FZB24 bei tropischem Feldgemüse und sein Wirkungsmechanismus." Zeitschrift für Pflanzenkrankheiten und Pflanzenschutz / Journal of Plant Diseases and Protection, vol. 108, no. 1, 2001, pp. 21-30. JSTOR, www.jstor.org/stable/43215378. Accessed 14 Dec. 2020.)(Hashem, Abeer & Tabassum, B. & Abd_Allah, Elsayed. (2019). Bacillus subtilis: A plant-growth promoting rhizobacterium that also impacts biotic stress. Saudi Journal of Biological Sciences. 26. 10.1016/j.sjbs.2019.05.004.)

Furthermore, bacterial spores were applied in the area of nanobiotechnology and building chemistry such as for self-healing concrete (crack healing), mortar stability and reduced water permeability [J.Y. Wang, H. Soens, W. Verstraete, N. De Belie, Self-healing concrete by use of microencapsulated bacterial spores, Cement and Concrete Research, Volume 56, 2014, 139-152, ISSN 0008-8846, https://doi.org/10.1016/j.cemconres.2013.11.009] [Ricca E, Cutting SM. Emerging Applications of Bacterial Spores in Nanobiotechnology. J Nanobiotechnology. 2003;1(1):6. Published 2003 Dec 15. doi:10.1186/1477-3155-1-6].

Additionally, bacterial spores were applied in the area of cleaning products, such as for cleaning of laundry, hard surfaces, sanitation and odor control (Caselli E. Hygiene: microbial strategies to reduce pathogens and drug resistance in clinical settings. Microb Biotechnol. 2017 Sep;10(5):1079-1083. doi: 10.1111/1751-7915.12755. Epub 2017 Jul 5) in the clinical and domestic setting. As an example, spores were used in cosmetic compositions such as skin cleaning products (US20070048244), for dishwashing agents (WO2014/107111), pipe degreasers (DE19850012), malodor control of laundry (WO2017/157778 and EP3430113) or the removal of allergens (US20020182184). Spores can also be embedded into non-biogenic matrices to catalyze its subsequent breakdown.

In addition, bacterial spores were applied in the area of human and animal nutrition and health. As an example different bacterial strains were applied to broilers as part of antibiotic replacement strategy (Neveling, D.P., Dicks, L.M. Probiotics: an Antibiotic Replacement Strategy for Healthy Broilers and Productive Rearing. Probiotics & Antimicro. Prot. 13, 1-11 (2021). https://doi.org/10.1007/s12602-020-09640-z). Other examples include aquaculture, pigs and many more (Nayak, S.K. (2021), Multifaceted applications of probiotic Bacillus species in aquaculture with special reference to Bacillus subtilis. Rev. Aquacult., 13: 862-906. https://doi.org/10.1111/raq.12503). Applications of bacterial spored for human health are also widely described (e.g. US 20180289752; Lee, NK., Kim, WS. & Paik, HD. Bacillus strains as human probiotics: characterization, safety, microbiome, and probiotic carrier. Food Sci Biotechnol 28, 1297-1305 (2019). https://doi.org/10.1007/s10068-019-00691-9).

The target substance can also be a fusaricidin, paeniserine, paeniprolixine or polymyxin. In each case it is particularly preferred that the one or more of the fusaricidins comprise any of fusaricidin A, B or D. As described below, fusaricidins, paeniserines, paeniproxilines or polymyxins have numerous applications particularly for promoting plant health and/or in plant pest control.

The target substance can also be a viscosity increasing or viscosifying substance, such as Paenan and/ or Levan. Microbial exopolysaccharides have numerous applications such as oil drilling, thickener in cosmetics, in paints, food, as rheology modifiers, in pharmaceuticals and in home care. Several applications are listed e.g. in Liyaskina EV, Rakova NA, Kitykina AA, Rusyaeva W, Toukach PV, Fomenkov A, Vainauskas S, Roberts RJ, Revin W. Production and characterization of the exopolysaccharide from strain Paenibacillus polymyxa 2020. PLoS One. 2021 Jul 6;16(7):e0253482. doi: 10.1371/journal.pone.0253482. PMID: 34228741; PMCID: PMC8259973.

Furthermore, the microorganism or spores thereof can be the target substance. As described e.g. by Grady EN, MacDonald J, Liu L, Richman A, Yuan ZC. Current knowledge and perspectives of Paenibacillus: a review. Microb Cell Fact. 2016 Dec 1;15(1):203. doi: 10.1186/s12934-016-0603-7. PMID: 27905924; PMCID: PMC5134293, many Paenibacillus species can promote crop growth directly via biological nitrogen fixation, phosphate solubilization, production of the phytohormone indole-3-acetic acid (IAA), and release of siderophores that enable iron acquisition. They can also offer protection against insect herbivores and phytopathogens, including bacteria, fungi, nematodes, and viruses.

The microorganism culture in step i) of the fermentation method of the present invention is preferably obtainable or obtained by
i.i) inoculating a pre-culture fermentation medium with a quantity of the microorganisms and
i.ii) cultivating the culture of step i.i) to provide a culture having
   a) the median cell corpuscular length is 0.8-2.9 µm, more preferably 1-2.5 µm, even more preferably 1.3-2 µm, even more preferably 1.3-1.8µm, and/or
   b) the 75th cell corpuscular length percentile is 1.3-3 µm, more preferably 1.3-2.9 µm, more preferably 1.5-2.5 µm, and/or
   c) the 25th cell corpuscular length percentile is 0.8-2.5 µm, more preferably 0.8-1.9 µm, more preferably 0.8-1.5 µm.

As described above, cells corpuscles having such cell corpuscular length are those belonging to a microorganism culture of the present invention, wherein the microorganisms of family Paenibacillaceae, preferably of genus Paenibacillus, are metabolically aligned and/or in an activated metabolic state. Also as described above, such microorganisms can also be described by other parameters, e.g. growth rate, cell size, viscosity, production of an indicator substance, optical density, cell count, course of pH, gas formation, oxygen uptake rate, CO2 transfer rate, and variance of any of these parameters.

The invention also provides a method for reducing fermentation time required for producing a desired amount of a target substance, and/or for reducing viscosity during fermentation, and/or for increasing fermentation repeat accuracy, comprising the steps of
i) providing, in a fermentation medium, a culture according to the invention, and
ii) cultivating the culture in the fermentation medium.

As described herein, in particular in the accompanying figures and examples, it is an advantage of a microorganism culture of the present invention that the microorganisms are in a metabolic state which allows to produce a target substance much faster than for those microorganisms growing according to the first growth type. Thus, the culture of the present invention, when used in a fermentation method, allows to produce a given amount of the target substance faster, or allows to produce more of the target substance within a given time. It is a particular advantage that such faster fermentation can be achieved while also realising a decrease in fermentation medium viscosity compared to a microorganism culture growing according to the first growth type.

Correspondingly, a microorganism culture of the present invention is preferably used to reduce the fermentation time required for producing a desired amount of a target substance, and/or to reduce viscosity during fermentation, and/or to increase fermentation repeat accuracy.

As described herein, the invention particularly improves reproducibility of fermentations using Paenibacillaceae microorganisms, preferably of genus Paenibacillus, and thus facilitates automated fermentations. Correspondingly the invention provides a cultivation parameter dataset for controlling a fermentation, comprising
- a designation of a set of applicable fermentation media,
- a designation of a set of applicable samples of microorganisms,
- a designation of fermentation conditions conducive to obtain a culture according to the invention by cultivating an applicable sample in an applicable fermentation medium, wherein the fermentation conditions preferably comprise at least one of: type of cultivation medium, amount of cultivation medium, desired cultivation temperature range, desired cultivation aeration parameter ranges, desired amount of produced cells, desired duration of cultivation, desired pH during cultivation, desired yield or amount of a target substance.

The cultivation parameter dataset advantageously can be used to steer a fermentation of Paenibacillaceae microorganisms, preferably of genus Paenibacillus. Due to the reproducibility obtainable by using a microorganism culture of the present invention, the cultivation parameter dataset is sufficient to steer such a fermentation to produce a target substance.

The invention correspondingly also provides a computer-implemented fermentation method, comprising
- receiving, by a computing device, a cultivation parameter dataset according to the present invention,
- receiving a fermentation medium conforming to the cultivation parameter dataset,
- receiving a volume of a sample of microorganisms conforming to the cultivation parameter dataset, and
- controlling, by the computing device, a fermenter to cultivate said microorganisms and/or spores thereof in the fermentation medium under the fermentation conditions of the cultivation parameter dataset.

As described herein, a fermentation according to the cultivation parameter dataset results in the production of a microorganism culture of the present invention and/or in maintaining such Paenibacillaceae microorganisms, preferably microorganisms of genus Paenibacillus, in a metabolically aligned and/or activated state, thereby securing the advantages of the present invention. In particular, the computer-implemented fermentation method allows to produce a target substance reliably, faster and/or in higher yield compared to microorganisms growing according to the first growth type.

Controlling preferably comprises
- determining one or more parameter indicative of metabolic activity, wherein the parameter is preferably selected from any of growth rate, cell size, viscosity, production of an indicator substance, optical density, cell count, course of pH, gas formation, oxygen uptake rate, CO2 transfer rate, and variance of any of the aforementioned parameters,
- comparing the one or more determined parameters with target fermentation conditions, preferably one or more parameters of the cultivation parameter dataset, and
- adjusting fermentation conditions to promote conformance of the one or more determined parameters with the said target fermentation conditions.

On a related note the invention also provides a method for producing one or more viscosity-increasing substances. The method comprises
i) inoculating a fermentation medium by a concentration of a microorganism culture according to the invention in a predetermined concentration sufficient to obtain, during cultivation, a desired viscosity, and
ii) cultivating the culture obtained in step i), and
iii) preferably recovering the one or more viscosity-increasing or viscosifying substances.

Thus, the method starts with a microorganism culture of the present invention. However, maintaining the cells in the second growth type leads to a reduction of viscosity during fermentation. Thus, the method typically involves a cultivation, in step ii), of a culture in a higher cell density. It is a particular advantage of the present invention that the microorganism culture of the present invention allows to improve reproducibility and yield of a target substance, including viscosity-increasing substances, even if the culture is only used for inoculating an industrial fermentation without maintaining conditions for growth according to the second growth type.

The invention is hereinafter further described by way of the accompanying figures and examples, neither of which is intended to limit the scope of the invention.

### EXAMPLES

### Example 1: Impact of shake flask inoculation volume on growth of P.polymyxa LU21132 (fig. 1)

The impact of the initial preculture inoculation volume on growth of P.polymyxa was investigated in shake flask cultivations using Paenibacillus strain LU21132.

Strain LU21132 is a polymyxin free mutant of wild-type isolate P.polymyxa LU17007 and derives from a random mutagenesis approach. The strain was exemplarily chosen to demonstrate impact of preculture inoculation volume in single- and two stage cultivations, but same phenomenon was also proven in wild-type strain P.polymyxa LU17007 and its further mutant successors such as LU20754 and LU21552 as well as in public Paenibacillus strains such as P.polymyxa DM365 or P.terrae DSM15891.

Different ratios (% v/v) from a working cell bank of strain LU21132 (0.1 - 4.0% v/v) were used for the inoculation of preculture flasks to analyze its impact on biomass formation of LU21132 within 24h of growth in PX-125 media. Tested inoculation volumes are listed in Table 1.

**Table 1: Shake flask inoculation volumes used to test impact of inoculation density on growth of P. polymyxa in 250ml shake flasks containing 30ml PX-125 media**

| inoculation vol. % [v/v] in culture media | ml of WCB used for inoculation of 30ml media |
|---|---|
| 0.1% | 0.03 |
| 0.2% | 0.06 |
| 0.4% | 0.12 |
| 0.6% | 0.18 |
| 0.8% | 0.24 |
| 1.0% | 0.3 |
| 2.0% | 0.6 |
| 4.0% | 1.2 |

WCB1041, used in this cultivation, was generated after 72h of cultivation in shake flask using a soy-based complex medium. Before filling, microscopic evaluation were assessed in order to identify the progress of sporulation within the culture. Under the microscope, spores as well as cells containing endospores were detected. Then WCB of the strain P.polymyxa LU21132 was filled up in 2ml cryo culture vials with 25% glycerol and 5% sucrose. The preparation procedure of all WCBs used in this study is listed in Table 5.

### Culture conditions

The composition of PX-125 is listed in Table 2. The components of the stock solution were dissolved in distilled water and either sterile filtered or autoclaved at 121 °C, 1 bar overpressure for 60 min. The sterile solutions were stored either at room temperature or at 4 °C. The antifoam agent was added to the main solution shortly before starting the autoclaving process. After mixing the stock solutions, the pH of the medium was set to 6.5 either with 25 % (w/w) ammonia solution or 40 % (w/w) phosphoric acid.

**Table 2: Composition of the complex medium PX-125 with the specification for storage (room temperature (RT) or 4 °C) and sterilization method (sterile-filtered / autoclaved, s/a) of the stock solution.**

| Stock solution | Component | Concentration in the medium [g/l] |
|---|---|---|
| Main solution (RT, a) | Citric acid monohydrate | 3.21 |
| | Dipotassium hydrogen phosphate | 1.00 |
| | Ammonium sulfate | 1.07 |
| | Magnesium sulfate heptahydrate | 1.62 |
| | Yeast extract | 5.00 |
| | Soy flour | 10.00 |
| | Antifoam | 0.2 |
| Calciumnitrate Tetrahydrate (4 °C, s) | Calcium nitrate tetrahydrate | 0.342 |
| Maltose (RT, a) | Maltose monohydrate | 63.00 |
| Vitamin solution (4 °C, s) | Thiamin hydrochloride | 0.005 |
| | Nicotinic acid | 0.005 |
| | Riboflavin | 0.0002 |
| | Biotin | 0.00005 |
| | Calcium pantothenate | 0.001 |
| | Pyridoxin hydrochloride | 0.005 |
| | Vitamin B12 | 0.00005 |
| | Lipoic acid | 0.00005 |
| Trace element solution (4 °C, s) | Manganese sulfate monohydrate | 0.013 |
| | Copper sulfate pentahydrate | 0.0046 |
| | Sodium molybdate dihydrate | 0.0028 |
| | Iron sulfate monohydrate | 0.015 |
| | Citric acid monohydrate | 0.4 |
| Water | | Add 1L |
| | **pH: 6.5** | |

Cultivation was conducted in 250ml shake flasks with baffles containing 30ml of culture media PX-125 sealed with breathable silicon plugs. Cultivation was performed at 33°C, 150 rpm and 25 mm shaking frequency for 24h.

To determine bacterial growth, optical density (OD) measurement at 600 nm was conducted using the corresponding non-inoculated medium as blank. The OD of the cultivation experiment with varying inoculation volumes is shown in figure 1.

### Example 2: Growth curve in MTP-scale cultivations at different inoculation volumes (fig. 2)

Cultivations of Paenibacillus LU20754 were performed in 48 well microtiter plates with flower geometry, sealed with a sterile membrane of m2p labs (F-GPR48-10, Gas-permeable sealing foil with evaporation reducing layer). The parameters for cultivations were 900 rpm shaking frequency, 3mm shaking diameter, 2.4mL total well volume and 1.2ml liquid volume per well, 85% humidity, 35°C. Biomass formation was assessed by scattered light analyses of each well at 620nm excitation and emission wavelength, respectively. Different inoculation ratios (0.6, 1.2 and 1.8% v/v) of a spore WCB (7.95E+07 spores/ml) of strain P. polymyxa LU20754 were used.

### Example 3: Cell size and cell arrangement (fig. 3 + 4) depending on inoculation volume

P. polymyxa strain LU21552 was cultivated in 1L shake flask w/o baffles using 100ml of a soy based medium. Two different inoculation volumes, 0.2% and 1% (v/v), respectively were tested using spore WCB1059 generated after 72h fermentation time (see table 5). Cultivation was carried out at 33°C, 250 rpm, 5cm throw. Light microscopic assessment was performed with a 1000x magnification after 28h cultivation.

The cell corpuscular length was determined by using the analyze particles function implemented in Fiji ( Schindelin J, Arganda-Carreras I, Frise E, Kaynig V, Longair M, Pietzsch T, Preibisch S, Rueden C, Saalfeld S, Schmid B, Tinevez JY, White DJ, Hartenstein V, Eliceiri K, Tomancak P, Cardona A: Fiji: an open-source platform for biological-image analysis. Nat Methods. 2012, 9: 676-682. DOI: 10.1038/nmeth.2019) and calculating the Feret's diameter of each corpuscle recognized by the particle analyzer (refer to images with particle outlines for selected cells). First, a threshold was applied to the RGB microscopy image using the default thresholding method to generate a binary image. Subsequently, the segmented image was used as input for the analyze particles function und all analyzed cells cell corpuscular lengths are displayed in the images showing the cell outlines as well as number in the analysis report. The following list shows the cell corpuscular lengths for each cell corpuscle identified in fig. 3A and 3B.

| Fig. 3B cell corpuscular lengths [µm] | | Fig. 3A cell corpuscular lengths [µm] | | | | | | | | | |
|---|---|---|---|---|---|---|---|---|---|---|---|
| 8.183 | 3 | 2.872 | 2.262 | 1.961 | 1.802 | 1.646 | 1.511 | 1.412 | 1.31 | 1.174 | 1.026 |
| 7.259 | 2.746 | 2.71 | 2.215 | 1.941 | 1.801 | 1.632 | 1.494 | 1.412 | 1.289 | 1.174 | 0.985 |
| 6.469 | 2.728 | 2.705 | 2.166 | 1.937 | 1.801 | 1.623 | 1.494 | 1.387 | 1.289 | 1.172 | 0.973 |
| 6.319 | 2.687 | 2.595 | 2.128 | 1.928 | 1.801 | 1.601 | 1.486 | 1.376 | 1.289 | 1.164 | 0.973 |
| 5.848 | 2.676 | 2.567 | 2.128 | 1.903 | 1.794 | 1.601 | 1.48 | 1.376 | 1.28 | 1.164 | 0.973 |
| 5.385 | 2.617 | 2.561 | 2.1 | 1.9 | 1.784 | 1.586 | 1.48 | 1.37 | 1.269 | 1.154 | 0.926 |
| 5.294 | 2.617 | 2.515 | 2.078 | 1.9 | 1.771 | 1.586 | 1.478 | 1.37 | 1.262 | 1.144 | 0.897 |
| 4.954 | 2.53 | 2.512 | 2.07 | 1.886 | 1.756 | 1.578 | 1.478 | 1.37 | 1.252 | 1.136 | 0.86 |
| 4.123 | 2.463 | 2.506 | 2.07 | 1.878 | 1.756 | 1.569 | 1.47 | 1.363 | 1.252 | 1.136 | 0.792 |
| 4.077 | 2.463 | 2.496 | 2.064 | 1.861 | 1.754 | 1.569 | 1.46 | 1.363 | 1.252 | 1.136 | |
| 4.033 | 2.415 | 2.481 | 2.064 | 1.861 | 1.754 | 1.569 | 1.451 | 1.361 | 1.216 | 1.12 | |
| 3.586 | 2.139 | 2.463 | 2.037 | 1.853 | 1.754 | 1.558 | 1.451 | 1.361 | 1.216 | 1.12 | |
| 3.523 | 1.978 | 2.415 | 2.032 | 1.853 | 1.754 | 1.556 | 1.445 | 1.361 | 1.216 | 1.12 | |
| 3.438 | | 2.409 | 2.031 | 1.849 | 1.729 | 1.548 | 1.437 | 1.343 | 1.216 | 1.101 | |
| 3.427 | | 2.386 | 2.024 | 1.828 | 1.699 | 1.527 | 1.431 | 1.343 | 1.216 | 1.093 | |
| 3.388 | | 2.343 | 2.024 | 1.824 | 1.689 | 1.517 | 1.418 | 1.328 | 1.216 | 1.069 | |
| 3.379 | | 2.343 | 2.013 | 1.811 | 1.687 | 1.515 | 1.418 | 1.323 | 1.177 | 1.069 | |
| 3.101 | | 2.309 | 2.006 | 1.811 | 1.68 | 1.515 | 1.418 | 1.323 | 1.177 | 1.046 | |
| 3.048 | | 2.295 | 1.985 | 1.802 | 1.68 | 1.515 | 1.414 | 1.314 | 1.174 | 1.046 | |
| 3.043 | | 2.264 | 1.97 | 1.802 | 1.646 | 1.511 | 1.414 | 1.31 | 1.174 | 1.046 | |

Thus, the cutoff cell corpuscular lengths in µm for the respective percentiles are:

| percentile | Fig. 3B | Fig. 3A |
|---|---|---|
| 0% (=minimum) | 2.0 | 0.8 |
| 25% (=25th cell corpuscular length percentile) | 2.7 | 1.3 |
| 50% (=median) | 3.4 | 1.5 |
| 75% (=75th cell corpuscular length percentile) | 4.1 | 1.9 |
| 100% (=maximum) | 8.2 | 2.9 |

### Example 4: impact of shake flask inoculation volume on fusaricidin production of P.polymyxa LU21132 (fig. 5)

Production of Fusaricidin was assessed in the cultivation of example 1 after 24h cultivation. For this, 50 µl of culture broth was mixed with 950 µl acetonitrile-water (1:1) mixture for extraction. The sample was treated for 30 min at 20°C in an ultrasonic bath. The sample then was centrifuged for 5 min at 14000 rpm and the supernatant filtered into a HPLC vial for measurement. Fusaricidin concentration was determined by HPLC-UV-VIS as listed in Table 3 and Table 4:

**Table 3: HPLC setting for quantification of Fusaricidin A, B and C in culture broth samples**

| | |
|---|---|
| Column: | Aqua C18, 250*4.6mm (Phenomenex) |
| Pre-column: | C18 Aqua |
| Temperature: | 40°C |
| Flowrate: | 1.00 mL/min |
| Injection volume: | 2.0 µL |
| Detection: | UV 200nm |
| Maximal pressure: | 400 bar |
| Stop time | 20 min |
| Eluent A: | H₂O with 0.1 % H3PO4 |
| Eluent B: | acetonitrile |

**Table 4: Solvent gradient for HPLC based quantification of Fusaricidin A, B and C in culture broth samples**

| Time [min] | A [%] | B [%] | Flow [ml/min] |
|---|---|---|---|
| 0.0 | 70.0 | 30.0 | 1.00 |
| 6.0 | 60.0 | 40.0 | 1.00 |
| 12.0 | 0.00 | 100.0 | 1.00 |
| 16.0 | 0.00 | 100.0 | 1.00 |
| 16.10 | 70.0 | 30 | 1.00 |

The production of Fusaricidin A, B and D in the cultivation of example 2 is shown in figure 2.

### Example 5: WCB inoculum density effect is independent of the WCB preparation procedure (fig. 6 + 7)

Varying working cell bank (WCB) preparation procedures were tested to identify the strain's sensitivity towards inoculation volumes in shake flask precultures. The WCB preparation procedures used are listed in Table 5.

**Table 5: WCB preparation protocols**

| **WCB #** | **Strain** | **preparation media** | **scale** | **Cult. time** | **cell type before harvest** | **cryo preservation** |
|---|---|---|---|---|---|---|
| *1003* | *LU20754* | *PX-48* | *1L bioreactor* | *48h* | *Spores* | *10% glycerol 5% sucrose -80°C* |
| 1041 Cryo | LU21132 | PX-99 | 1L shake flask | 72h | cells w/ endospores, free spores | 25% glycerol 5% sucrose -80°C |
| 1041 Lyo | LU21132 | | | | | freeze drying of culture broth, RT |
| 1048 | LU21132 | PX-105 | 1L bioreactor | 48h | viable cells w/ and w/o endospores | 25% glycerol, 5% sucrose, -80°C |
| 1049 | LU21132 | PX-125 | | | | |
| 1050 | LU21132 | PX-105 | | 72h | cells w/ endospores, free spores | |
| 1051 | LU21132 | PX-125 | | | | |
| *1059* | *LU21552* | *PX-162* | *1L bioreactor* | *72h* | *Spores* | *25% glycerol, 5% sucrose, -80°C* |

0.3% and 0.6% (v/v, see Table 1) of six different WCBs from strain LU21132 (see Table 5) were taken to start a shake flask cultivation. WCB vials were heated at 60°C for 30min before inoculation of the preculture media PX-125. Cultivation was carried out in 250ml shake flask according to the procedure of example 1.

Bacterial growth, as evaluated by OD600 analyses, was assessed after 24h of cultivation and is shown in figure 6. Furthermore, production of the secondary metabolite Fusaricidin was quantified after 24h of growth using the method described in example 2. The level of Fusaricidin as a function of the WCB inoculation volume is shown in figure 7.

### Example 6: Preculture inoculum volume does affect Fusaricidin titer in main culture (two stage process) (fig. 8)

After 24h cultivation of the precultures in example 3, 600µl of each approach was transferred into main culture medium PX-143 in order to investigate the effect of preculture inoculation volume on the performance in a two-stage process.

Main culture medium: PX-143

**Table 6: Composition of the complex medium PX-143 with the specification for storage (room temperature (RT) or 4 °C) and sterilization method (sterile-filtered / autoclaved, s / a) of the stock solution.**

| | Component | Concentration in the medium [g/l] |
|---|---|---|
| Main solution (RT, a) | Citric acid monohydrate | 3.21 |
| | Dipotassium hydrogen phosphate | 1.00 |
| | Ammonium sulfate | 1.07 |
| | Magnesium sulfate heptahydrate | 1.62 |
| | Yeast extract | 10.00 |
| | Soy flour | 13.00 |
| | Antifoam | 0.2 |
| Calciumnitrate Tetrahydrate (4 °C, s) | Calcium nitrate tetrahydrate | 0.342 |
| Maltose (RT, a) | Maltose syrup (50%) | 156.00 |
| Vitamin solution (4 °C, s) | Thiamine hydrochloride | 0.005 |
| | Nicotinic acid | 0.005 |
| | Riboflavin | 0.0002 |
| | Biotin | 0.00005 |
| | Calcium pantothenate | 0.001 |
| | Pyridoxine hydrochloride | 0.005 |
| | Vitamin B12 | 0.00005 |
| | Lipoic acid | 0.00005 |
| Trace element solution (4 °C, s) | Manganese sulfate monohydrate | 0.013 |
| | Copper sulfate pentahydrate | 0.0046 |
| | Sodium molybdate dihydrate | 0.0028 |
| | Iron sulfate monohydrate | 0.015 |
| | Citric acid monohydrate | 0.4 |
| Buffer (4 °C, s) | 2-(N-morpholino)ethanesulfonic acid | 100mM |
| Water | | Add 1L |
| | **pH: 6.5** | |

Once again, production of Fusaricidin in main cultures was assessed after 48h of cultivation using the method described in example 2. The Fusaricidin levels detected in the cultivation of example 4 are shown in figure 5. Here, the fusaricidin level of the main cultures which were inoculated with the preculture flasks having 0.6% preculture inoculation volume are shown relative to the fusaricidin level detected in the parallel main cultures from the 0.3% preculture inoculation variant.

### Example 6: Preculture inoculum volume does synchronize growth in main culture (two stage process) of various WCB (fig. 9)

Biomass formation in the main cultures of example 5 was analyzed by OD600 measurements of final culture broth samples after 48h of cultivation. Figure 6 shows the range of the OD600 [A.U.] values from the cultivation using the six WCBs from Table 5 at 0.3% and 0.6% inoculation volume. Cultivation conditions: 250ml shake flasks w/ baffles, 30ml media, 150 rpm, 2.5cm throw, 33°C, pH 6.5, 24h preculture, 48h main culture.

### Example 7: Main culture inoculation volume influences culture broth viscosity (fig. 10)

Impact of inoculation volume on culture broth viscosity was analyzed in soy-based exopolysaccharide production medium (table 7)

**Table 7: Composition of the exopolysaccharide production medium with the specification for storage (room temperature (RT) or 4 °C) and sterilization method (sterile filtered / autoclaved, s / a) of the stock solutions.**

| | Component | Concentration in the medium [g/l] |
|---|---|---|
| Main solution (RT, a) | Dipotassium hydroqen phosphate | 1.67 |
| | Calcium chloride dihydrate | 0.05 |
| | Magnesium sulfate heptahydrate | 1.33 |
| | Soy peptone | 5 |
| Glucose (RT, a) | Glucose monohydrate | 50 |
| Vitamin solution (4 °C, s) | Thiamine hydrochloride | 0.005 |
| | Nicotinic acid | 0.005 |
| | Riboflavin | 0.0002 |
| | Biotin | 0.00005 |
| | Calcium pantothenate | 0.001 |
| | Pyridoxine hydrochloride | 0.005 |
| | Vitamin B12 | 0.00005 |
| | Lipoic acid | 0.00005 |
| Trace element solution (4 °C, s) | Manganese sulfate monohydrate | 0.013 |
| | Copper sulfate pentahydrate | 0.0046 |
| | Sodium molybdate dihydrate | 0.0028 |
| | Iron sulfate monohydrate | 0.015 |
| | Citric acid monohydrate | 0.4 |
| Buffer (4 °C, s) | 2-(N-morpholino)ethanesulfonic acid | 100mM |
| Water | | Add 1L |
| | **pH: 7** | |

Cultivation of P.polymyxa LU21132 was performed in 250 ml shake flasks w/ baffles, filled with 30ml media (table 7) at 150 rpm, 2.5cm throw, 33°C and pH 6.5. Viscosity was assessed after 16h cultivation time with an Anton Paar rheometer, type MCR302 via rotary rheology using a cylindrical geometry. The measurements are carried out at T = 33 °C. At the beginning, the samples were preconditioned in a pre-shear experiment at constant shear rate of 10 s⁻¹ for 100 s. 10 data points are recorded (every 10 s). After preconditioning, viscosity is measured as a function of the shear rate. Therefore, the shear rate is varied from 1 s⁻¹ to 100 s⁻¹. 25 data points are recorded logarithmically. As indicated by OD600, EPS formation is in part decoupled from growth, with a special emphasis on 2, 4 and 6% inoculation volume.

## Claims

1. Microorganism culture, wherein the microorganism is of family Paenibacillaceae, preferably of genus Paenibacillus, wherein the cells in the culture are
- metabolically aligned and/or
- in an activated metabolic state.

2. Culture according to claim 1, wherein
a) the median cell corpuscular length is 0.8-2.9 µm, more preferably 1-2.5 µm, even more preferably 1.3-2 µm, even more preferably 1.3-1.8µm, and/or
b) the 75th cell corpuscular length percentile is 1.3-3 µm, more preferably 1.3-2.9 µm, more preferably 1.5-2.5 µm, and/or
c) the 25th cell corpuscular length percentile is 0.8-2.5 µm, more preferably 0.8-1.9 µm, more preferably 0.8-1.5 µm.

3. Culture according to claim 1 or 2, obtainable or obtained by inoculating a cultivation medium with a concentration of microorganisms sufficient to produce said culture, wherein the concentration is preferably determined by
i) cultivating a series of cultures inoculated with different inoculation concentrations for a sufficient time to differentiate cultures by a parameter indicative of metabolic activity, wherein the parameter is preferably selected from any of growth rate, cell size, viscosity, production of an indicator substance, optical density, cell count, course of pH, gas formation, oxygen uptake rate, CO2 transfer rate, and variance of any of the aforementioned parameters, and
ii) determining said one or more parameter, and
iii) selecting an inoculation concentration based on the determined one or more parameters.

4. Fermentation method, comprising
i) providing a fermentation broth comprising a microorganism culture of any of claims 1-3 in a fermentation medium, and
ii) cultivating the fermentation broth under conditions suitable to produce a target substance, and
iii) preferably recovering the target substance.

5. Fermentation method of claim 4, wherein the microorganism culture in step i) is obtainable or obtained by
i.i) inoculating a pre-culture fermentation medium with a quantity of the microorganisms and
i.ii) cultivating the culture of step i.i) to provide
a) the median cell corpuscular length is 0.8-2.9 µm, more preferably 1-2.5 µm, even more preferably 1.3-2 µm, even more preferably 1.3-1.8µm, and/or
b) the 75th cell corpuscular length percentile is 1.3-3 µm, more preferably 1.3-2.9 µm, more preferably 1.5-2.5 µm, and/or
c) the 25th cell corpuscular length percentile is 0.8-2.5 µm, more preferably 0.8-1.9 µm, more preferably 0.8-1.5 µm.

6. Fermentation method according to any of claims 4-5, wherein the target substance is selected from
- microorganisms, preferably viable cells and/or spores,
- one or more metabolic products, preferably at least one fusaricidin, paeniserine, paeniprolixine or polymyxin, preferably at least two or more fusaricidins, paeniserines, paeniproxilines or polymyxins, more preferably 3- 120 fusaricidins, wherein the one or more fusaricidins comprise any of fusaricidin A, B or D, and/or surfactin and/or iturin.

7. Method for reducing fermentation time required for producing a desired amount of a target substance, and/or for reducing viscosity during fermentation, and/or for increasing fermentation repeat accuracy, comprising the steps of
i) providing, in a fermentation medium, a culture according to any of claims 1-3, and
ii) cultivating the culture in the fermentation medium.

8. Use of a culture according to any of claims 1-3 for reducing fermentation time required for producing a desired amount of a target substance, and/or for reducing viscosity during fermentation, and/or for increasing fermentation repeat accuracy.

9. Cultivation parameter dataset for controlling a fermentation, comprising
- a designation of a set of applicable fermentation media,
- a designation of a set of applicable samples of microorganisms,
- a designation of fermentation conditions conducive to obtain a culture according to any of claims 1-3 by cultivating an applicable sample in an applicable fermentation medium, wherein the fermentation conditions preferably comprise at least one of: type of cultivation medium, amount of cultivation medium, desired cultivation temperature range, desired cultivation aeration parameter ranges, desired amount of produced cells, desired duration of cultivation, desired pH during cultivation, desired yield or amount of a target substance.

10. Computer-implemented fermentation method, comprising
- receiving, by a computing device, a cultivation parameter dataset according to claim 9,
- receiving a fermentation medium conforming to the cultivation parameter dataset,
- receiving a volume of a sample of microorganisms conforming to the cultivation parameter dataset, and
- controlling, by the computing device, a fermenter to cultivate said microorganisms and/or spores thereof in the fermentation medium under the fermentation conditions of the cultivation parameter dataset.

11. Computer-implemented fermentation method according to claim 10, wherein controlling comprises
- determining one or more parameter indicative of metabolic activity, wherein the parameter is preferably selected from any of growth rate, cell size, viscosity, production of an indicator substance, optical density, cell count, course of pH, gas formation, oxygen uptake rate, CO2 transfer rate, and variance of any of the aforementioned parameters,
- comparing the one or more determined parameters with target fermentation conditions, and
- adjusting fermentation conditions to promote conformance of the one or more determined parameters with the said target fermentation conditions.

12. Method for producing one or more viscosity-increasing substances, comprising
i) inoculating a fermentation medium by a concentration of a microorganism culture according to any of claims 1-3 in a predetermined concentration sufficient to obtain, during cultivation, a desired viscosity, and
ii) cultivating the culture obtained in step i), and
iii) preferably recovering the one or more viscosity-increasing substances.
